# EUROPEAN PATENT APPLICATION

(11) **EP 3 825 330 A1**
(43) Date of publication of application: **26.05.2021**
(21) Application number: 19306488.8
(22) Date of filing: 19.11.2019
(51) Int. Cl.: C07K 16/28, C07K 16/46

(54) **ANTI-CD117 ANTIBODIES AND METHODS OF USE THEREOF**

(71) Applicant: International-Drug-Development-Biotech, 69007 Lyon (FR)
(72) Inventor: VERMOT-DESROCHES, Claudine, 69570 Dardilly (FR); BRESSON, Corinne, 69570 Dardilly (FR); ROUSSET, Carine, 38110 Sainte Blandine (FR); VUILLERMOZ, Boris, 69380 Les Cheres (FR); SUBIGER, Olivier, 69220 Belleville (FR)
(74) Representative: Lavoix

(57) **Abstract**

The invention relates to the fields of immunology, oncology, and more specifically, to antibodies that can be used in the treatment of various cancers, more particularly cancers expressing CD117 antigen. These antibodies or antigen-binding molecules may be monoclonal antibodies or binding fragments thereof, they may be in particular chimeric or humanized. These antibodies may be antagonistic. The invention also relates to anticancerous or pharmaceutical compositions containing an antibody according to this invention and methods of use of these, either for laboratory work or for medical treatment.

## Description

### FIELD OF THE INVENTION

The present invention relates to the fields of immunology, oncology, and more specifically, to antibodies or antibody fragment (i.e. antigen-binding fragment) thereof, specifically binding CD117, that can be used in the treatment of various cancers. Cancers targeted by the invention are expressing CD117 antigen. These antibodies may be monoclonal antibodies or antibody fragments thereof, specifically binding CD117, they may be in particular chimeric or humanized. These antibodies and their antigen-binding fragments may be antagonistic. The invention also relates to anticancerous or pharmaceutical compositions containing an antibody or antigen-binding fragment according to this invention and methods of use of these, either for laboratory work or for medical treatment.

### BACKGROUND OF THE INVENTION

Stem cell factor receptor (c-Kit or CD117) exerts multiple biological effects on target cells upon binding its ligand stem cell factor (SCF). KIT is a cell surface tyrosine kinase receptor whose SCF ligand triggers homodimerization and activation of downstream effector pathways involved in cell survival, proliferation, homing, or differentiation. Aberrant activation of c-Kit results in dysregulated signaling and is implicated in the pathogenesis of numerous cancers. The overexpression of SCF and/or KIT is also observed in a number of cancers, including 50% of AML and small cell lung cancer. Furthermore SCF stimulates c-Kit tyrosine kinase activity and downstream signaling pathways such as mitogen-activated protein kinase (MAPK) and protein kinase B (AKT), in addition to reducing tumor cell line growth in vitro.

KIT-activating mutations are major oncogenic drivers in subsets of acute myeloid leukemia (AML), in mast cell leukemia, and in gastrointestinal stromal tumors (GIST). The use of tyrosine kinase inhibitors (TKi as for example imatinib) in these pathologies is however hampered by initial or acquired resistance following treatment. The development of more specific and effective c-Kit or CD117 therapies is warranted given its essential role in tumorigenesis such as human small cell lung carcinoma (SCLC), melanoma, and leukemia tumor cell lines.

Currently, a great deal of attention has focused on the development of novel immunotherapy strategies for the treatment of cancer. One such strategy is antibody-based cancer therapy. Using antibody, the targeting of membrane KIT/CD117 extracellular immunoglobulin domains implicated in KIT ligand binding and/or Kit homodimerization could inhibit KIT-dependent growth of leukemic cell lines expressing KIT or constitutively active KIT mutants and/or Kit resistant cancers.

There is still a need for antibodies that may represent efficient therapeutic tools for therapeutic application in particular for treating human malignancies harboring c-Kit (CD117) receptor and/or to bypass TKI resistance of certain KIT mutants.

### SUMMARY OF INVENTION

The present invention thus relates to antigen-binding molecules or antibodies specifically binding to CD117. Such an antigen-binding molecule or antibody may be a full-size monoclonal antibody specifically binding to CD117 or a CD117-binding fragment thereof. A murine monoclonal antibody called R036-05 has been selected which binds to CD117 and exhibits interesting and unique properties rendering it very advantageous for an anticancerous treatment. From this murine antibody, the applicant and a specialist as well may derive and produce antigen (CD117)-binding molecules that keep or substantially keep the specific set of CDRs of the murine antibody and/or the same or substantially same binding ability. This allows the person skilled in the art, based on the following description and its general knowledge to prepare antibodies "derived from" this murine monoclonal antibody, especially antibodies that are more suited to the treatment of a given mammal species, especially a human. As it will be apparent from the following, the terms "derived from" may encompass different CD117-binding molecules retaining or substantially retaining the antigen-binding properties of the parental murine monoclonal antibody, and the Applicant has successfully generated powerful chimeric and humanized antibodies.

The antibodies according to the invention comprise CDR sequences, or VH and VL sequences comprising CDR sequences. The CDR sequences may be defined in accordance with IMGT, Kabat, Chotia or the Common numbering system (CNS, which retain the common sequence between IMGT and Kabat). In particular, the anti-CD117 antibodies of the invention may comprise L-CDR 1, 2 and 3 and/or H-CDR 1, 2 and 3 as follows (Table 1):

| | SEQ ID NO: | IMGT | SEQ ID NO: | Kabat | SEQ ID NO: | Chotia | SEQ ID NO: | CNS |
|---|---|---|---|---|---|---|---|---|
| VH mR036.05 | | | | | | | | |
| H-CDR1 | 1 | GYTFTRY W | 6 | RYWMN | 11 | GYTFTRY | | RYW |
| H-CDR2 | 2 | IDPSDSEI | 7 | RIDPSDSEI HYNQNFKD | 12 | DPSDSE | 2 | IDPSDSEI |
| H-CDR3 | 3 | ARDYGS DYYAMD Y | 8 | DYGSDYYA MDY | 8 | DYGSDYY AMDY | 8 | DYGSDYY AMDY |

| VL mR036.05 | | | | | | | | |
|---|---|---|---|---|---|---|---|---|
| L-CDR1 | 4 | SSVSY | 9 | SASSSVSY MH | 9 | SASSSVSY MH | 4 | SSVSY |
| L-CDR2 | | STS | 10 | STSNLTS | 10 | STSNLTS | | STS |
| L-CDR3 | 5 | HQWSSY P | 5 | HQWSSYP | 5 | HQWSSYP | 5 | HQWSSYP |

The antigen-binding molecules or antibodies of the invention may thus be, in particular, any antibodies, monoclonal antibodies, chimeric antibodies, humanized antibodies, or any binding fragment of them, comprising the sets of H-CDRs and L-CDRs. A "humanized antibody" or "chimeric antibody" shall mean an antibody derived from the parent murine antibody by the methods available to the skilled person and for example, those disclosed herein. Preferably, a "humanized antibody" or "chimeric antibody", or a binding fragment thereof, will comprise the set of 6 CDRs of the murine antibody R036-05 or a variant version of these CDRs especially in the case of the humanized antibody. All these antibodies, such as in particular the humanized or chimeric antibodies, and the antibody fragments, retain or substantially retain the antigen-binding properties of the parental murine antibody R036-05. The disclosure of the VH and VL sequences and set of CDRs of the parental murine antibody is also deemed allowing the skilled person developing full human antibodies and bispecific antibodies. The CDRs or some of them may differ from the murine CDRs following SDR approaches (supergrafting). The present invention thus discloses variant H-CDR1 and L-CDR1 (see Table 2), and these variants are those present in the herein disclosed humanized monoclonal antibodies or binding fragments thereof for which the H-CDR2, H-CDR3, L-CDR2 and L-CDR-3 are identical to those presented in Table 1.

Thus, the anti-CD117 antibodies of the invention may comprise L-CDR 1, 2 and 3 and/or H-CDR 1, 2 and 3 as follows (Table 1):

| | SEQ ID NO: | IMGT | SEQ ID NO: | Kabat | SEQ ID NO: | Chotia | SEQ ID NO: | CNS |
|---|---|---|---|---|---|---|---|---|
| VH mR036.05 | | | | | | | | |
| H-CDR1 | 33 | GYTFTSY W | 34 | SYWMN | 35 | GYTFTSY | | SYW |
| H-CDR2 | 2 | IDPSDSEI | 7 | RIDPSDSEI HYNQNFKD | 12 | DPSDSE | 2 | IDPSDSEI |
| H-CDR3 | 3 | ARDYGS DYYAMD Y | 8 | DYGSDYYA MDY | 8 | DYGSDYY AMDY | 8 | DYGSDYY AMDY |

| VL mR036.05 | | | | | | | | |
|---|---|---|---|---|---|---|---|---|
| L-CDR1 | 36 | QSVSY | 37 | SASQSVSY MH | 37 | SASQSVSY MH | 36 | QSVSY |
| L-CDR2 | | STS | 10 | STSNLTS | 10 | STSNLTS | | STS |
| L-CDR3 | 5 | HQWSSY P | 5 | HQWSSYP | 5 | HQWSSYP | 5 | HQWSSYP |

Advantageously, the antibodies or CD117-binding molecules according to the invention are antagonists or antagonist antibodies. These antagonistic antibodies are capable of, directly or indirectly, substantially counteracting, reducing or inhibiting the biological activity CD117 exerts or induces when activated or when bound to its ligand, especially its ligand SCF.

The invention thus primarily concerns an antibody, preferably monoclonal antibody, or a fragment thereof, said antibody or fragment thereof specifically binding to CD117 and comprising (based on Tables 1 and 2)
- (1) a VH chain comprising the following three CDRs: a H-CDR1 of sequence SEQ ID NO: 1, a H-CDR2 of sequence SEQ ID NO: 2, a H-CDR3 of sequence SEQ ID NO: 3; and a VL chain comprising the following three CDRs: a L-CDR1 of sequence SEQ ID NO: 4, a L-CDR2 of sequence STS, a L-CDR3 of sequence SEQ ID NO: 5;
- (2) a VH chain comprising the following three CDRs: a H-CDR1 of sequence SEQ ID NO: 33, a H-CDR2 of sequence SEQ ID NO: 2, a H-CDR3 of sequence SEQ ID NO: 3; and a VL chain comprising the following three CDRs: a L-CDR1 of sequence SEQ ID NO: 36, a L-CDR2 of sequence STS, a L-CDR3 of sequence SEQ ID NO: 5;
- (3) a VH chain comprising the following three CDRs: a H-CDR1 of sequence SEQ ID NO: 6, a H-CDR2 of sequence SEQ ID NO: 7, a H-CDR3 of sequence SEQ ID NO: 8; and a VL chain comprising the following three CDRs: a L-CDR1 of sequence SEQ ID NO: 9, a L-CDR2 of sequence SEQ ID NO: 10, a L-CDR3 of sequence SEQ ID NO: 5;
- (4) a VH chain comprising the following three CDRs: a H-CDR1 of sequence SEQ ID NO: 34, a H-CDR2 of sequence SEQ ID NO: 7, a H-CDR3 of sequence SEQ ID NO: 8; and a VL chain comprising the following three CDRs: a L-CDR1 of sequence SEQ ID NO: 37, a L-CDR2 of sequence SEQ ID NO: 10, a L-CDR3 of sequence SEQ ID NO: 5;
- (5) a VH chain comprising the following three CDRs: a H-CDR1 of sequence SEQ ID NO: 11, a H-CDR2 of sequence SEQ ID NO: 12, a H-CDR3 of sequence SEQ ID NO: 8; and a VL chain comprising the following three CDRs: a L-CDR1 of sequence SEQ ID NO: 9, a L-CDR2 of sequence SEQ ID NO: 10, a L-CDR3 of sequence SEQ ID NO: 5;
- (6) a VH chain comprising the following three CDRs: a H-CDR1 of sequence SEQ ID NO: 35, a H-CDR2 of sequence SEQ ID NO: 12, a H-CDR3 of sequence SEQ ID NO: 8; and a VL chain comprising the following three CDRs: a L-CDR1 of sequence SEQ ID NO: 37, a L-CDR2 of sequence SEQ ID NO: 10, a L-CDR3 of sequence SEQ ID NO: 5;
- (7) a VH chain comprising the following three CDRs: a H-CDR1 of sequence RYW, a H-CDR2 of sequence SEQ ID NO: 2, a H-CDR3 of sequence SEQ ID NO: 8; and a VL chain comprising the following three CDRs: a L-CDR1 of sequence SEQ ID NO: 4, a L-CDR2 of sequence STS, a L-CDR3 of sequence SEQ ID NO: 5; or
- (8) a VH chain comprising the following three CDRs: a H-CDR1 of sequence SYW, a H-CDR2 of sequence SEQ ID NO: 2, a H-CDR3 of sequence SEQ ID NO: 8; and a VL chain comprising the following three CDRs: a L-CDR1 of sequence SEQ ID NO: 36, a L-CDR2 of sequence STS, a L-CDR3 of sequence SEQ ID NO: 5.

The invention particularly concerns an antibody, preferably monoclonal antibody, or fragment thereof, said antibody or fragment thereof specifically binding to CD117 and comprising a VH region of sequence SEQ ID NO: 13; a VL region of sequence SEQ ID NO: 15; or preferably a VH region of sequence SEQ ID NO: 13 and a VL region of sequence SEQ ID NO: 15. These regions are those of the murine antibody chR036.05, and these regions comprise the sets of CDRs disclosed in Table 1 and in paragraphs (1), (3), (5) and (7) above.

In an embodiment according to the invention, the antibodies defined herein in the preceding or following paragraphs, are intended for use in treating cancer expressing CD117.

The antibody, preferably monoclonal antibody, according to the invention may be a chimeric antibody. In particular, it comprises a human Fc region, preferably an IgG1 human Fc region, linked to the VH region as disclosed herein (to form the Heavy Chain) and/or (preferably and) a human Kappa region, linked to the VL region as disclosed herein (to form the Light Chain). In an embodiment, the Fc region is the native Fc, especially the native human IgG1 Fc, which means that the Fc region does not comprise substitutions of one or more Fc amino acid. As explained thereafter however, the Fc may be modified to confer specific effector functions to the antibody.

Thus, the antibody, preferably monoclonal antibody, according to the invention comprises a Heavy Chain comprising a VH chain according to paragraphs (1), (2), (3), (4), (5), (6), (7) or (8) above, and a human Fc region, preferably an IgG1 human Fc region; and/or (preferably and) a Light Chain comprising a VL chain according to paragraphs (1), (2), (3), (4), (5), (6), (7) or (8) above, and a human Kappa region.

The invention thus concerns an antibody, preferably monoclonal antibody, or a fragment thereof, said antibody or fragment thereof specifically binding to CD117 and comprising
- (1) a Heavy Chain comprising a VH region comprising the following three CDRs: a H-CDR1 of sequence SEQ ID NO: 1, a H-CDR2 of sequence SEQ ID NO: 2, a H-CDR3 of sequence SEQ ID NO: 3, and a human Fc region, preferably an IgG1 human Fc region; and a Light Chain comprising the following three CDRs: a VL region comprising a L-CDR1 of sequence SEQ ID NO: 4, a L-CDR2 of sequence STS, a L-CDR3 of sequence SEQ ID NO: 5, and a human Kappa region;
- (2) a Heavy Chain comprising a VH region comprising the following three CDRs: a H-CDR1 of sequence SEQ ID NO: 33, a H-CDR2 of sequence SEQ ID NO: 2, a H-CDR3 of sequence SEQ ID NO: 3, and a human Fc region, preferably an IgG1 human Fc region; and a Light Chain comprising the following three CDRs: a VL region comprising a L-CDR1 of sequence SEQ ID NO: 36, a L-CDR2 of sequence STS, a L-CDR3 of sequence SEQ ID NO: 5, and a human Kappa region;
- (3) a Heavy Chain comprising a VH region comprising the following three CDRs: a H-CDR1 of sequence SEQ ID NO: 6, a H-CDR2 of sequence SEQ ID NO: 7, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 9, a L-CDR2 of sequence SEQ ID NO: 10, a L-CDR3 of sequence SEQ ID NO: 5, and a human Kappa region;
- (4) a Heavy Chain comprising a VH region comprising the following three CDRs: a H-CDR1 of sequence SEQ ID NO: 34, a H-CDR2 of sequence SEQ ID NO: 7, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 37, a L-CDR2 of sequence SEQ ID NO: 10, a L-CDR3 of sequence SEQ ID NO: 5, and a human Kappa region;
- (5) a Heavy Chain comprising a VH region comprising the following three CDRs: a H-CDR1 of sequence SEQ ID NO: 11, a H-CDR2 of sequence SEQ ID NO: 12, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 9, a L-CDR2 of sequence SEQ ID NO: 10, a L-CDR3 of sequence SEQ ID NO: 5, and a human Kappa region;
- (6) a Heavy Chain comprising a VH region comprising the following three CDRs: a H-CDR1 of sequence SEQ ID NO: 35, a H-CDR2 of sequence SEQ ID NO: 12, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 37, a L-CDR2 of sequence SEQ ID NO: 10, a L-CDR3 of sequence SEQ ID NO: 5, and a human Kappa region;
- (7) a Heavy Chain comprising a VH region comprising the following three CDRs: a H-CDR1 of sequence RYW, a H-CDR2 of sequence SEQ ID NO: 2, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 4, a L-CDR2 of sequence STS, a L-CDR3 of sequence SEQ ID NO: 5, and a human Kappa region; or
- (8) a Heavy Chain comprising a VH region comprising the following three CDRs: a H-CDR1 of sequence SYW, a H-CDR2 of sequence SEQ ID NO: 2, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 36, a L-CDR2 of sequence STS, a L-CDR3 of sequence SEQ ID NO: 5, and a human Kappa region.

In an embodiment, the invention also concerns an antibody, preferably monoclonal antibody, or a fragment thereof, said antibody or fragment thereof specifically binding to CD117 and comprising:
- a VH region comprising a sequence with 85, 90, 95, 96, 97, 98, 99 or 100% identity after optimal alignment with sequence SEQ ID NO: 13, and a VL region comprising a sequence with 85, 90, 95, 96, 97, 98, 99 or 100% identity after optimal alignment with sequence SEQ ID NO: 15;
- a VH region comprising a sequence with 85, 90, 95, 96, 97, 98, 99 or 100% identity after optimal alignment with sequence SEQ ID NO: 25, and a VL region comprising a sequence with 85, 90, 95, 96, 97, 98, 99 or 100% identity after optimal alignment with sequence SEQ ID NO: 27;
- a VH region comprising a sequence with 85, 90, 95, 96, 97, 98, 99 or 100% identity after optimal alignment with sequence SEQ ID NO: 29, and a VL region comprising a sequence with 85, 90, 95, 96, 97, 98, 99 or 100% identity after optimal alignment with sequence SEQ ID NO: 27;
- a VH region comprising a sequence with 85, 90, 95, 96, 97, 98, 99 or 100% identity after optimal alignment with sequence SEQ ID NO: 31, and a VL region comprising a sequence with 85, 90, 95, 96, 97, 98, 99 or 100% identity after optimal alignment with sequence SEQ ID NO: 27.

The invention particularly concerns a chimeric antibody, preferably chimeric monoclonal antibody, comprising a chimeric Heavy Chain of sequence SEQ ID NO: 21 and a chimeric Light Chain of sequence SEQ ID NO: 23.

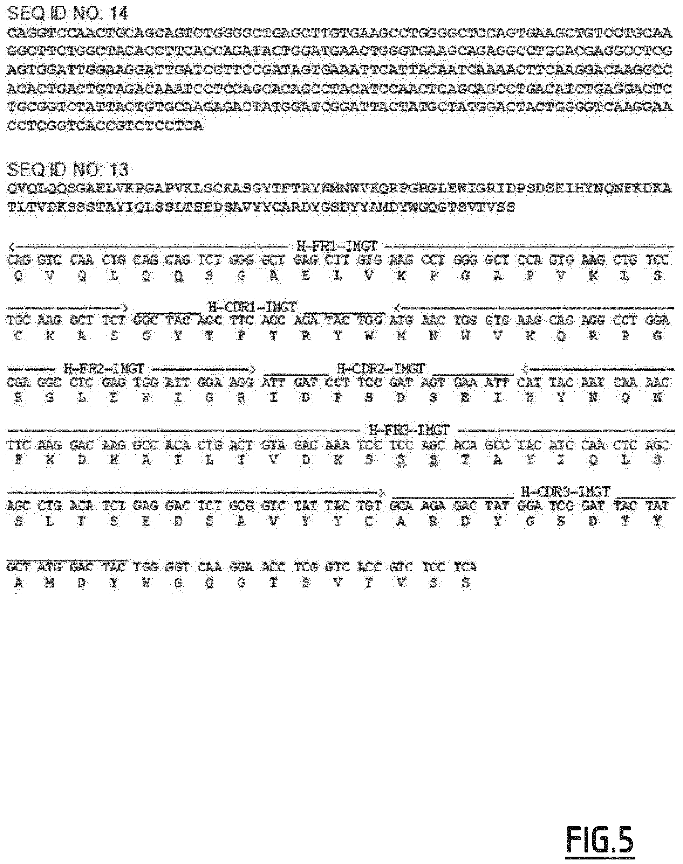

The VH and VL regions may comprise the sets of respective three CDRs as disclosed herein, and Framework Regions FR1, FR2, FR3 and FR4. In an embodiment, the Framework Regions are those of the murine parental antibody R036-05, as represented on Figure 5.

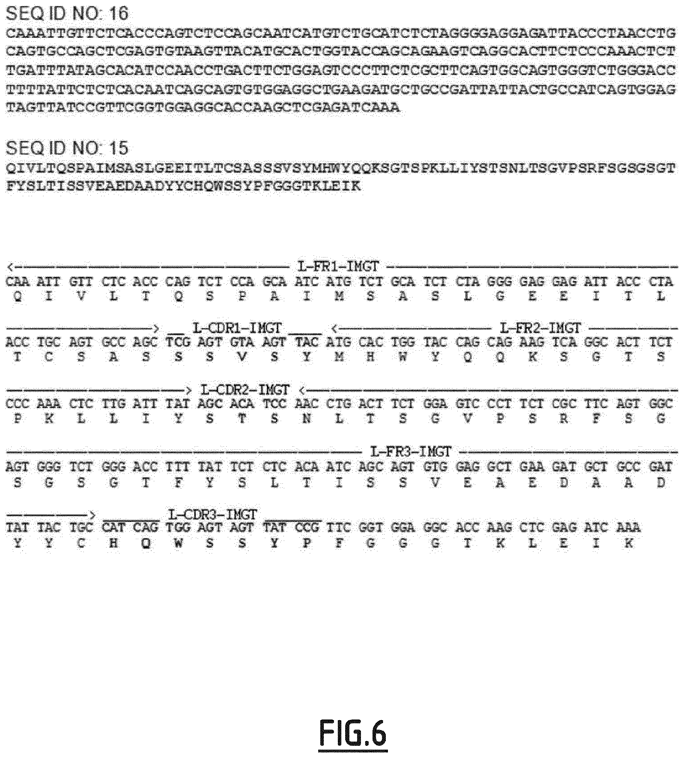

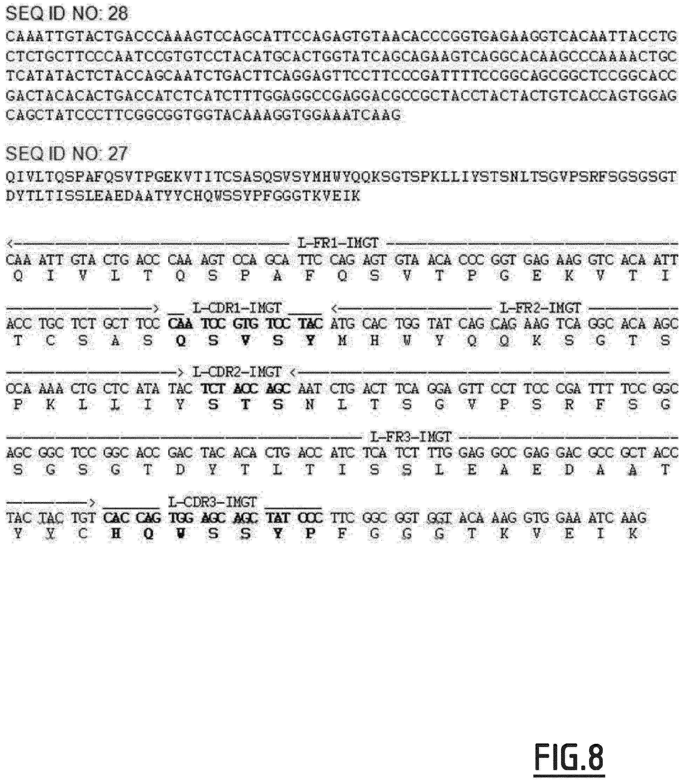

In another embodiment, there can be changes of some amino acids in the Framework Regions (FR) and these changes are made so as the antigen-binding properties are not changed or substantially changed. Thus, the invention also concerns an antibody, preferably monoclonal antibody, or a fragment thereof, said antibody or fragment thereof specifically binding to CD117 and comprising
- a VH region comprising the H-CDR1, H-CDR2 and H-CDR3 according to IMGT, Kabat, Chotia, or CNS (Table 1 or Table 2) and Framework Regions FR1, FR2, FR3 and FR4 wherein at least one of the Framework Regions FR1, FR2, FR2 and FR4 thereof has a sequence with at least 85%, 90%, 95%, 96%, 97%, 98% or 99% of identity after optimal alignment with the respective FR1, FR2, FR3 and FR4 sequence of Figure 5 (or SEQ ID NO: 13), Figure 7 (or SEQ ID NO: 25), Figure 9 (or SEQ ID NO: 29), Figure 10 (or SEQ ID NO: 31) and
- a VL region comprising the L-CDR1, L-CDR2 and L-CDR3 according to IMGT, Kabat, Chotia, or CNS (Table 1 or Table 2) and Framework Regions FR1, FR2, FR3 and FR4 wherein at least one of the Framework Regions FR1, FR2, FR2 and FR4 thereof has a sequence with at least 85%, 90%, 95% 96%, 97%, 98% or 99% of identity after optimal alignment with the respective FR1, FR2, FR3 and FR4 sequence of Figure 6 (or SEQ ID NO: 15) or Figure 8 (or SEQ ID NO: 27).

Examples of such variants are disclosed in this application with 3 humanized monoclonal antibodies.

The present invention thus also concerns a humanized antibody, preferably a humanized monoclonal antibody, or a fragment thereof, said humanized antibody or fragment thereof specifically binding to CD117 and comprising
(i) a VH of sequence SEQ ID NO: 25 and a VL of sequence SEQ ID NO: 27;
(ii) a VH of sequence SEQ ID NO: 29 and a VL of sequence SEQ ID NO: 27;
(iii) a VH of sequence SEQ ID NO: 31 and a VL of sequence SEQ ID NO: 27;
(iv) a VH of sequence SEQ ID NO: 25, 29 or 31; or
(v) a VL of sequence SEQ ID NO: 27.

Preferably, the humanized antibodies as those disclosed in the examples further comprise a human Fc region, especially an IgG1 human Fc region, and/or a human Kappa region. In an embodiment, the Fc region is the native Fc, especially the native human IgG1 Fc. As explained thereafter, the Fc may be modified to confer specific effector functions to the antibody.

Thus, the invention further concerns a humanized antibody, preferably a monoclonal antibody, specifically binding to CD117 and comprising:
(i) a Heavy Chain comprising a VH region of sequence SEQ ID NO: 25 and a human Fc region, preferably an IgG1 human Fc region; and a Light Chain comprising a VL region of sequence SEQ ID NO: 27 and a human Kappa region;
(ii) a Heavy Chain comprising a VH region of sequence SEQ ID NO: 29 and a human Fc region, preferably an IgG1 human Fc region; and a Light Chain comprising a VL region of sequence SEQ ID NO: 27 and a human Kappa region;
(iii) a Heavy Chain comprising a VH region of sequence SEQ ID NO: 31 and a human Fc region, preferably an IgG1 human Fc region; and a Light Chain comprising a VL region of sequence SEQ ID NO: 27 and a human Kappa region;
(iv) a Heavy Chain comprising a VH region of sequence SEQ ID NO: 25, 29 or 31 and a human Fc region, preferably an IgG1 human Fc region;
(v) a Light Chain comprising a VL region of sequence SEQ ID NO: 27 and a human Kappa region.

The antibodies of the invention may be full-size monoclonal antibodies or bispecific antibodies comprising a VH region and a VL region as disclosed hereinabove.

The fragments of antibody may be selected from: a F(ab')2, a Fab, a Fab', a dsFv, a (dsFv)2, a scFv, a sc(Fv)2, a diabody, a triabody, a minibody, a nanobody, a scFv-Fc, and a multimeric scFv.

Another object of the invention is a pharmaceutical composition or an anticancerous composition comprising such an antibody or fragment thereof, as disclosed herein, and a pharmaceutically acceptable vehicle or diluent.

The herein-disclosed antibodies, preferably monoclonal antibodies, and the fragments thereof, specifically binding to CD117, and the pharmaceutical compositions or anticancerous compositions comprising at least one the herein-disclosed antibodies, preferably monoclonal antibodies, and the fragments thereof, when used or upon administration to a subject in need thereof, may:
- inhibit and/or reverse CD117-ligand binding, especially CD117/SCF binding;
- inhibit CD117-expressing cancer cell proliferation or growth;
- have a specific or unique pattern of activity on the c-Kit phosphorylation compared to other anti-CD117 antibodies; in particular, by binding to CD117 the antibodies of the invention do not up-modulate stem-cell factor induced Y730 (Tyr730) phosphorylated CD117; more particularly, the antibodies of the invention down-modulate stem-cell factor induced Y719 (Tyr719) phosphorylated CD117 and do not up-modulate stem-cell factor induced Y730 (Tyr730) phosphorylated CD117;
- inhibit both SCF ligand binding and SCF induced cell proliferation; and/or
- specifically recognize a conformational epitope; conformational epitopes may particularly be found at the surface of these cancer cells and this renders the antibodies of the invention particularly interesting for cancer treatment.

The present invention also relates to such CD117-binding molecules or antibodies and pharmaceutical or anticancerous compositions for use in treating cancer, especially cancer expressing CD117 or cancer comprising cells expressing CD117.

The present invention also concerns a herein-disclosed antibody, preferably monoclonal antibody, or the fragment thereof, specifically binding to CD117, or a pharmaceutical or anticancerous composition comprising a herein-disclosed antibody, preferably monoclonal antibody, or the fragment thereof, for use in treating a cancer, especially a cancer expressing CD117 or cancer comprising cells expressing CD117. This use may further comprise:
- inhibiting and/or reversing CD117-ligand binding, especially CD117/SCF binding;
- inhibiting CD117-expressing cancer cell proliferation or growth; and/or
- inducing cell death of CD117 positive cancer cells; and/or
- inhibiting both SCF ligand binding and SCF induced cell proliferation; and/or
- having a specific or unique pattern of activity on the c-Kit phosphorylation compared to other anti-CD117 antibodies; and/or
- recognizing a conformational epitope.

The present invention also relates to the use of such antibodies for the manufacture of a pharmaceutical or anticancerous composition for treating cancer, especially cancer expressing CD117 or cancer comprising cells expressing CD117.

The present invention also relates to a method of treatment of cancer, especially cancer expressing CD117 or cancer comprising cells expressing CD117, comprising administering to a patient in need thereof a sufficient amount of such an antibody or such a pharmaceutical or anticancerous composition.

Other objects of the present invention will appear in the following detailed description.

### DETAILED DESCRIPTION

### Definitions

The term "antibody" is used in the broadest sense and specifically covers intact monoclonal antibodies, and derived antibodies and antibody fragments as disclosed herein, so long as they exhibit the desired biological activity, as defined herein.

"Native antibodies" and "native immunoglobulins" are usually glycoproteins of about 150,000 daltons, composed of two identical Light (L) Chains and identical Heavy (H) Chains. Each Light Chain is linked to a Heavy Chain by one covalent disulfide bond, while the number of disulfide linkages varies among the Heavy Chains of different immunoglobulin isotypes. Each Heavy and Light chain also has regularly spaced intrachain disulfide bridges. Each Heavy Chain has at one end a variable domain (VH or V_{H}). Each Light Chain has at one end a variable domain (VL or V_{L}).

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes. Light Chains are classified as either kappa or lambda. Heavy Chains are classified as gamma, mu, alpha, delta, or epsilon, which in turn define the immunoglobulin classes, IgG, IgM, IgA, IgD and IgE, respectively.

With respect to antibodies of the invention, the term "specifically binds" refers to antibodies that bind to one or more epitopes of CD117, but which do not substantially recognize and bind other molecules in a sample containing a mixed population of antigenic biological molecules.

"Antibody fragments" comprise a portion of an intact antibody, which comprises at least the antigen-binding or variable region of the intact antibody. A suitable "antibody fragment" is a fragment of antibody that has the capability to bind to the CD117 binding region or epitope specifically recognized by the murine parental 36-05 antibody and keep or substantially keep the antigen-binding property of the intact 36-05 antibody (parental murine or derived form thereof, i.e. chimeric or humanized). More particularly, the fragments keep or substantially keep the biological property of the intact (full-size) antibody. The fragment is capable of, directly or indirectly, substantially counteracting, reducing or inhibiting CD117 biological activity resulting from CD117 activation. Optionally, the fragment is capable of neutralizing the biological activity resulting from CD117 activation or formation of a complex between CD117 and its ligand, such as SCF. The fragment preferably comprises the 6 CDRs or the VH and VL regions of the parent antibody (murine, chimeric, humanized, etc. antibody) and, as mentioned earlier, may be in particular a F(ab')₂, Fab, Fab', dsFv, (dsFv)₂, scFv, sc(Fv)₂, a diabody, a triabody, a minibody, a nanobody, a scFv-Fc, or a multimeric scFv.

The terms "polypeptide", "peptide" and "protein" are used interchangeably herein to refer to a polymer of amino acid residues. The terms apply to naturally occurring amino acid polymers as well as to amino acid polymers in which one or more amino acid residues is an artificial chemical analogue of a corresponding naturally occurring amino acid. The term also includes variants on the traditional peptide linkage joining the amino acids making up the polypeptide. Preferred "peptides", "polypeptides", and "proteins" are chains of amino acids whose carbons are linked through peptide bonds. Peptides also include essentially any polyamino acid including, but not limited to peptide mimetics such as amino acids joined by ether as opposed to an amine bond.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated, in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies *(Kabat et al., NIH Publ. 1991; No. 91-3242, Vol. 1, 647-669).* The constant domains are not involved directly in binding an antibody to an antigen, but exhibit various effectors functions, such as participation of the antibody in antibody-dependent cellular toxicity.

The monoclonal antibodies herein specifically include "chimeric" antibodies (immunoglobulins) in which a portion of the Heavy and/or Light Chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567; Morrison et al., Proc. Natl. Acad. Sci. USA, 81:6851-6855 (1984)).

Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S. L et al., Proc. Natl. Acad. Sci. USA 1984; 81:6851-6855*;* U.S. Pat. No. 5,202,238 and U.S. Pat. No. 5,204,244. WO 2006/093794 relates to heterodimeric protein binding compositions. WO 99/37791 describes multipurpose antibody derivatives. Morrison et al., the J. Immunolog. 1998; 160:2802-2808 refers to the influence of variable region domain exchange on the functional properties of IgG.

"Humanized" forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementarity-determining region (CDR) of the recipient are replaced by residues from a CDR of a non-human species (donor antibody) which is mouse herein, having the desired specificity, affinity, and capacity.

In a preferred embodiment, a murine CDR is grafted into the framework region of a human antibody to prepare the "humanized antibody". See, e.g., Riechmann, L. et al., Nature. 1988; 332: 323-327*;* and Neuberger, MS et al., Nature. 1985; 314: 268-270*.* Particularly preferred CDRs correspond to those representing sequences recognizing the antigens noted above for chimeric antibodies.

Other forms of "chimeric antibodies" or "humanized antibodies" encompassed by the present invention are those in which the constant region has been additionally modified or changed from that of the original antibody to generate the properties according to the invention, especially in regard to C1q binding and/or Fc receptor (FcR) binding.

Furthermore, humanized antibodies may comprise residues that are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature. 1986; 321:522-525*;* Reichmann et al., Nature. 1988; 332:323-329*; and* Presta et al., Curr. Op. Struct. Biol. 1992; 2:593-596*.*

Humanized antibodies, or antibodies adapted for non-rejection by other mammals, may be produced using several technologies such as resurfacing and CDR grafting. As used herein, the resurfacing technology uses a combination of molecular modeling, statistical analysis and mutagenesis to alter the non-CDR surfaces of antibody variable regions to resemble the surfaces of known antibodies of the target host. Antibodies can be humanized using a variety of other techniques including CDR-grafting (EP0239400; WO91/09967; U.S. Patent Nos. 5,530,101 and 5,585,089), veneering or resurfacing (EP0592106; EP0519596; Padlan (1991) Molecular Immunology 28(4/5):489-498; Studnicka et al. (1994) Protein Engineering 7(6):805-814; Roguska et al. (1994) Proc. Natl. Acad. Sci U.S.A. 91:969-973), and chain shuffling (U.S. Patent No. 5,565,332). Human antibodies can be made by a variety of methods known in the art including phage display methods. See also U.S. Patent Nos. 4,444,887, 4,716,111, 5,545,806, and 5,814,318; and International patent application WO98/46645, WO98/50433, WO98/24893, WO98/16654, WO96/34096, WO96/33735, and WO91/10741.

The mechanism of action of MAbs is complex and appears to vary for different MAbs. There are multiple mechanisms by which MAbs cause target cell death. These include apoptosis, CDC, ADCC and inhibition of signal transduction.

"Treatment" or "therapy" refer to both therapeutic treatment and prophylactic or preventative measures. Preferably, it is therapeutic treatment.

"Mammal" for purposes of treatment or therapy refers to any animal classified as a mammal, including humans, domestic and farm animals, and zoo, sports, or pet animals, such as dogs, horses, cats, cows, etc. Preferably, the mammal is human.

The terms "cancer" and "cancerous" refer to or describe the physiological condition in mammals that is typically characterized by unregulated cell growth.

The term "nucleic acid" or "oligonucleotide" or grammatical equivalents herein refer to at least two nucleotides covalent linked together. A nucleic acid of the present invention is preferably single-stranded or double stranded and will generally contain phosphodiester bonds.

Amino acid sequence "variants" (or mutants) of the antibody are prepared by introducing appropriate nucleotide changes into the antibody DNA, or by nucleotide synthesis. Such modifications can be performed, however, only in a very limited range, e.g. as described herein. For example, the modifications do not alter the above mentioned antibody characteristics such as the IgG isotype and antigen-binding, but may improve the yield of the recombinant production, protein stability or facilitate the purification.

A "variant" of a molecule is a sequence that is substantially similar to the sequence of the native molecule. For nucleotide sequences, variants include those sequences that, because of the degeneracy of the genetic code, encode the identical amino acid sequence of the native protein. Naturally occurring allelic variants such as these can be identified with the use of well-known molecular biology techniques, as, for example, with polymerase chain reaction (PCR) and hybridization techniques. Variant nucleotide sequences also include synthetically derived nucleotide sequences, such as those generated, for example, by using site-directed mutagenesis that encode the native protein, as well as those that encode a polypeptide having amino acid substitutions. Generally, nucleotide sequence variants of the invention will have in at least one embodiment 40%, 50%, 60%, to 70%, e.g., 71%, 72%, 73%, 74%, 75%, 76%, 77%, 78%, to 79%, generally at least 80%, e.g., 81%-84%, at least 85%, e.g., 86%, 87%, 88%, 89%, 90%, 91%, 92%, 93%, 94%, 95%, 96%, 97%, 98% or 99%, sequence identity to the native (endogenous) nucleotide sequence.

The term "inhibit" refers to a decrease in an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 95, 100%, or any amount of reduction in between as compared to native or control levels.

### Antibodies

In an embodiment, the antibody specifically binding to CD117 comprises one or two, preferably two, binding domains comprising a pair of VH and VL chains comprising the six CDRs of murine Monoclonal antibody (R36-05, or Mab 36-05), as disclosed in Table 1 *supra,* or of Humanized Hz-36 antibodies, as disclosed in Table 2 *supra.* Preferably, each one of these antibodies comprise two identical VH and VL chains as disclosed.

Each one of these antibodies binds specifically to an epitope or a binding region on the CD117 receptor, and as disclosed herein, the epitope or binding region is deemed conformational. The CDRs in Table 1 originate from a murine antibody. These antibodies according to the invention may be fully murine and this is a first series of embodiments. In other embodiments, these antibodies are chimeric antibodies wherein part of the antibody is human and more particularly the murine VH and VL regions are combined to human sequences to complete the whole Heavy and Light Chains, and retain or substantially retain the antigen-binding properties of the parental murine antibody. In another embodiment, these antibodies are humanized antibodies, wherein these humanized antibodies or binding fragments thereof comprise the same set of 6 CDRs in their respective VH and VL regions with humanized framework (FR) regions, and retain or substantially retain the antigen-binding properties of the parental murine antibody. In still another embodiment, these humanized antibodies or binding fragments thereof further comprise some mutations or amino acid changes in at least one CDR of the set of six. As an example, these humanized antibodies or binding fragments thereof comprise the 6 CDRs disclosed in Table 2, wherein H-CDR1 and L-CDR1 comprise amino acid changes with respect to the murine ones (Table 1).

The following Table 3 is the "Sequence Information Table" regrouping the list of relevant amino acid and corresponding nucleic acid sequences in addition to the CDRs sequences disclosed *supra.*

| Identification | Amino Acids | Nucleotid es | SEQ ID NO: |
|---|---|---|---|
| Murine VH | X | | 13 |
| Murine VH | | X | 14 |
| Murine VL | X | | 15 |
| Murine VL | | X | 16 |
| Human IgG1 Fc | X | | 17 |
| Human IgG1 Fc | | X | 18 |
| Human Kappa | X | | 19 |
| Human Kappa | | X | 20 |
| Chimeric Heavy Chain (murine VH + human IgG1 Fc) | X | | 21 |
| Chimeric Heavy Chain (murine VH + human IgG1 Fc) | | X | 22 |
| Chimeric Light Chain (murine VL + human Kappa) | X | | 23 |
| Chimeric Light Chain (murine VL + human Kappa) | | X | 24 |
| Humanized VH (H-36.5Hz27) | X | | 25 |
| Humanized VH (H-36.5Hz27) | | X | 26 |
| Humanized VL (L-36.5Hz31) | X | | 27 |
| Humanized VL (L-36.5Hz31) | | X | 28 |
| Humanized VH (H-36.5Hz30) | X | | 29 |
| Humanized VH (H-36.5Hz30) | | X | 30 |
| Humanized VH (H-36.5Hz31) | X | | 31 |
| Humanized VH (H-36.5Hz31) | | X | 32 |

In an embodiment, the antibody comprises a human IgG, especially human IgG1 Fc region. Based on this disclosure, the skilled person is able to modify an antibody according to the invention to comprise a variant human optimized IgG Fc region, preferably IgG1 Fc region, wherein this variant region comprises one or more amino acid substitutions, so as to modulate PDC, ADCC and/or CDC.

### Compositions

Another object of the invention is a composition or a pharmaceutical composition comprising at least one anti-CD117 antibody or fragment thereof, as disclosed above and provided herein. The composition may further comprise a vehicle or diluent, in particular a vehicle or diluent suited to the intended use of the antibody. If the composition is a pharmaceutical composition, this composition comprises said anti-CD117 antibody or CD117-binding molecule and a pharmaceutically acceptable carrier, diluent or excipient.

The pharmaceutical compositions may comprise at least one anti-CD117 antibody or fragment thereof, specifically binding to CD117, according to the invention, and at least one additional antibody directed against another target and/or at least one chemotherapeutic drug (such as small molecule), for a simultaneous, separate or sequential administration with antibody(ies) of the invention, to a mammal, including man. As additional active principle, one may cite doxorubicine, gemcitabine, camptothecin, paclitaxel.

The other target may be selected from the group consisting of another epitope on CD117, another cancer marker or receptor, and a combination thereof. Thus in a particular embodiment, the pharmaceutical composition may comprise an anti-CD117 antibody or CD117-binding fragment thereof according to the invention, and another anti-CD117 antibody or antigen-binding fragment thereof.

Pharmaceutically acceptable carriers or excipients that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat.

The pharmaceutical compositions of the present invention may be administered orally, parenterally, by inhalation spray, topically, rectally, nasally, buccally, vaginally or via an implanted reservoir. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The pharmaceutical compositions of this invention may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added. Alternatively, the compositions of this invention may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols. The compositions of this invention may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used. The compositions of this invention may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

For example, an antibody present in a pharmaceutical composition of this invention can be supplied at a concentration of 10 mg/mL in either 100 mg (10 mL) or 500 mg (50 mL) single-use vials. The product may be formulated for IV administration in 9.0 mg/mL sodium chloride, 7.35 mg/mL sodium citrate dihydrate, 0.7 mg/mL polysorbate 80, and Sterile Water for Injection. The pH may be adjusted to 6.5.

An exemplary suitable dosage range for an antibody in a pharmaceutical composition of this invention may be between about 1 mg/m² and 500 mg/m². However, it will be appreciated that these schedules are exemplary and that an optimal schedule and regimen can be adapted taking into account the affinity and tolerability of the particular antibody in the pharmaceutical composition that must be determined in clinical trials. A pharmaceutical composition of the invention for injection (e.g., intramuscular, i.v.) could be prepared to contain a pharmaceutically acceptable carrier, diluent or excipient, e.g. sterile buffered water (e.g. 1 ml for intramuscular), and between about 1 ng to about 100 mg, e.g. about 50 ng to about 30 mg or more preferably, about 5 mg to about 25 mg, of anti-CD117 antibody or CD117-binding molecule according to the invention.

In certain embodiments, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded in vivo. Biodegradable polyalkyl-cyanoacrylate nanoparticles that meet these requirements are contemplated for use in the present invention, and such particles may be are easily made. Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

### Methods of use

The antibodies or pharmaceutical compositions according to the invention are intended for use as a medicament, especially against cancer, in particular cancers expressing CD117 or cancer having cancerous cells expressing CD117. The present invention thus relates to a method of treating such a cancer in a subject in need thereof (mammal, preferably human) comprising administering to the subject a sufficient or therapeutically effective amount of an antibody of the present invention or an antigen-binding fragment thereof, or a composition according to the invention.

In an aspect, the method leads to an inhibition of growth of a tumor cell expressing CD117.

In an aspect, the method leads to an inhibition and/or reversion of CD117-ligand binding, especially CD117/SCF binding, resulting in the inhibition of cancer cell proliferation or growth.

The invention also aims at treating cancers associated with CD117 expression or overexpression, preferentially cancer, preferably leukemia, lymphoma or solid cancers. More particular examples of such cancers include myeloid leukemia, squamous cell cancer, small-cell lung cancer, non-small cell lung cancer, gastrointestinal cancer, renal cancer, pancreatic cancer, glioblastoma, cervical cancer, ovarian cancer, liver cancer, bladder cancer, hepatoma, breast cancer, colon cancer, colorectal cancer, endometrial carcinoma, salivary gland carcinoma, kidney cancer, prostate cancer, vulval cancer, thyroid cancer, hepatic carcinoma and various types of head and neck cancer. Examples are specifically those listed cancers expressing CD117 or having cancerous cells expressing CD117.

The invention also relates to a method of treating a cancer expressing CD117 in a subject having TKI sensitive or resistance comprising administering to the subject a sufficient or therapeutically effective amount of an antibody of the present invention or an antigen-binding fragment thereof, or a composition according to the invention.

The present invention also relates to a method of treatment comprising administering to a patient in need thereof a sufficient amount of such an antibody or such a pharmaceutical or anticancerous composition in order to
- inhibit and/or reverse CD117-ligand, especially CD117/SCF binding;
- inhibit CD117-expressing cancer cell proliferation or growth;
- inhibit CD117 signaling leading to inhibition of signaling activation and/or cell activation;
- induce cell death of CD117 positive cancer cells;
- inhibit both SCF ligand binding and SCF induced cell proliferation; and/or
- recognize a conformational epitope.

Our experimental data clearly demonstrate an innovative profile of the antibodies of the invention inhibiting both SCF ligand binding and SCF induced cell proliferation with a specific pattern of activity on the c-Kit phosphorylation compared to other anti-CD117 antibodies.

In each of the embodiments of the use and treatment methods described herein, the anti-CD117-binding antibody or molecule may be delivered in a manner consistent with conventional methodologies associated with management of the disease or disorder for which treatment is sought. In accordance with the disclosure herein, an effective amount of the CD117-binding antibody or molecule is administered to a patient in need of such treatment for a time and under conditions sufficient to treat the disease or disorder.

As used herein, the terms "treatment" and "treat" refer to curative or disease modifying treatment, including treatment of subjects who have cancer or have been diagnosed as suffering from cancer, especially CD117-expressing cancer, and includes suppression of clinical relapse. The treatment may be administered to a subject having a cancer, in order to cure, delay the onset of, reduce the severity of, or ameliorate one or more symptoms of said cancer, or in order to prolong the survival of a subject beyond that expected in the absence of such treatment.

By "therapeutic regimen" is meant the pattern of treatment of cancer, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of cancer. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of cancer, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "therapeutically effective amount" or "effective amount" refers to an amount effective, at dosages and for periods of time necessary, to achieve a desired therapeutic result. A therapeutically effective amount of the antibody of the present invention or an antigen-binding fragment thereof may vary according to factors such as the disease state, age, sex, and weight of the individual, and the ability of the antibody of the present invention or an antigen-binding fragment thereof to elicit a desired response in the individual. A therapeutically effective amount is also one in which any toxic or detrimental effect of the antibody or of an antigen-binding fragment thereof are outweighed by the therapeutically beneficial effects. The efficient dosages and dosage regimens for the antibody of the present invention or an antigen-binding fragment thereof depend on the disease or condition to be treated and may be determined by the persons skilled in the art. A physician having ordinary skill in the art may readily determine and prescribe the effective amount of the pharmaceutical composition required. For example, the physician could start doses of the antibody of the present invention or an antigen-binding fragment thereof employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. Typically, the ability of a compound to inhibit cancer may, for example, be evaluated in an animal model system predictive of efficacy in human tumors. Alternatively, this property of a composition may be evaluated by examining the ability of the compound to induce cytotoxicity by in vitro assays known to the skilled practitioner. A therapeutically effective amount of said antibody or an antigen-binding fragment thereof may decrease tumor size, or otherwise ameliorate symptoms in a subject. One of ordinary skill in the art would be able to determine such amounts based on such factors as the subject's size, the severity of the subject's symptoms, and the particular composition or route of administration selected.

The disclosed antibodies or antigen-binding fragments thereof can be administered as a therapeutic agent in amounts ranging from about 0.001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 0.5 mg/kg to about 25 mg/kg, or from about 0.5 to about 10, 5, 3 or 2 mg/kg of subject body weight per day, one or more times a day, to obtain the desired therapeutic effect. The desired dosage may be delivered three times a day, two times a day, once a day, every other day, every third day, every week, every two weeks, every three weeks, or every four weeks. In certain embodiments, the desired dosage may be delivered using multiple administrations (e.g., two, three, four, five, six, seven, eight, nine, ten, eleven, twelve, thirteen, fourteen, or more administrations). Administration may e.g. be intravenous, intramuscular, intraperitoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some embodiments, the efficacy of the treatment is monitored during the therapy, e.g. at predefined points in time. In some embodiments, the efficacy may be monitored by visualization of the disease area, or by other diagnostic methods described further herein, e.g. by performing one or more PET-CT scans, for example using a labeled antibody of the present invention, or an antigen-binding fragment thereof. If desired, an effective daily dose of a pharmaceutical composition may be administered as two, three, four, five, six or more sub-doses administered separately at appropriate intervals throughout the day, optionally, in unit dosage forms. In some embodiments, the monoclonal antibodies of the present invention are administered by slow continuous infusion over a long period, such as more than 24 hours, in order to minimize any unwanted side effects. An effective dose of an antibody of the present invention may also be administered using a weekly, biweekly or triweekly dosing period. The dosing period may be restricted to, e.g., 8 weeks, 12 weeks or until clinical progression has been established. As non-limiting examples, treatment according to the present invention may be provided as a daily dosage of an antibody of the present invention or an antigen-binding fragment thereof in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

In certain embodiments, an anti-CD117 antibody or an antigen-binding fragment thereof according to the invention is used in combination with a second agent for treatment of a disease or disorder. When used for treating cancer, an antibody of the invention may be used in combination with conventional cancer therapies such as, e.g., surgery, radiotherapy, chemotherapy, or combinations thereof.

### Combination

The present invention also provides for therapeutic applications or methods of treatment where an antibody of the present invention or an antigen-binding fragment thereof is used in combination with at least one further therapeutic agent, e.g. for treating cancer. Such administration may be simultaneous, separate or sequential. For simultaneous administration the agents may be administered as one composition or as separate compositions, as appropriate. The further therapeutic agent is typically relevant for the disorder to be treated. Exemplary therapeutic agents include other anti-cancer antibodies, cytotoxic agents, chemotherapeutic agents, anti-angiogenic agents, anti-cancer immunogens, cell cycle control/apoptosis regulating agents, hormonal regulating agents, and other agents described below.

In some embodiments, the antibody of the present invention or an antigen-binding fragment thereof is used in combination with a chemotherapeutic agent. The term "chemotherapeutic agent" refers to chemical compounds that are effective in inhibiting tumor growth.

Targeting CD117 with the antibodies of the present invention or an antigen-binding fragment thereof in combination with existing chemotherapeutic treatments will be more effective in killing the tumor cells than chemotherapy alone. A wide variety of drugs have been employed in chemotherapy of cancer. Examples include, but are not limited to, cisplatin, taxol, etoposide, mitoxantrone, actinomycin D, campthotecin, methotrexate, gemcitabine, mitomycin, dacarbazine, 5-fluorouracil, doxorubicine and daunomycin.

In one approach, the CD117-binding antibody or molecule is added to a standard chemotherapy regimen, in treating a cancer patient. For those combinations in which the antibody or an antigen-binding fragment thereof and additional anti-cancer agent(s) exert a synergistic effect against cancer cells, the dosage of the additional agent(s) may be reduced, compared to the standard dosage of the second agent when administered alone. The antibody or an antigen-binding fragment thereof may be co-administered with an amount of an anti-cancer drug that is effective in enhancing sensitivity of cancer cells to the antibody combination.

In one method of the invention, the CD117-binding antibody or molecule is administered to the patient prior to administration of a second anti-cancer agent. One alternative method comprises administering the second anti-cancer agent prior to administering the CD117-binding antibody or molecule and second agent on an alternative schedule. In another embodiment, the CD117-binding antibody or molecule and second agent are administered simultaneously.

The method of the invention may provide for the inclusion in a therapeutic regimen involving the use of at least one other treatment method, such as irradiation, chemotherapy with small molecule or antibody. The method of the invention may directly include the administration of a sufficient amount of at least one additional antibody directed against another target and/or at least one chemotherapeutic drug (such as small molecule), for a simultaneous, separate or sequential administration with antibody(ies) of the invention, or an antigen-binding fragment thereof, to a mammal, including man. As additional active principle, one may cite doxorubicine, gemcitabine, camptothecin, paclitaxel or the other drugs mentioned above. In an embodiment, lung cancer and breast cancer is treated using such combination. This combination more generally is useful for cancers (in particular aggressive cancers) which do not respond well to treatment with the drug alone or the antibodies/antibody of the invention or antigen-binding fragment thereof alone, and for which the combination leads to a synergistic effect.

### Production of antibodies

The antibodies of the present invention and antigen-binding fragments thereof are produced by any technique known in the art, such as, without limitation, any chemical, biological, genetic or enzymatic technique, either alone or in combination. Typically, knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, antibodies of the present invention can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

Mammalian cells are the preferred hosts for production of therapeutic antibodies, due to their capability to glycosylate proteins in the most compatible form for human applications. Bacteria very rarely glycosylate proteins, and like other type of common hosts, such as yeasts, filamentous fungi, insect and plant cells yield glycosylation patterns associated with rapid clearance from the blood stream.

Among mammalian cells, Chinese hamster ovary (CHO) cells are the most commonly used. In addition to giving suitable glycosylation patterns, these cells allow consistent generation of genetically stable, highly productive clonal cell lines. They can be cultured to high densities in simple bioreactors using serum-free media, and permit the development of safe and reproducible bioprocesses. Other commonly used animal cells include baby hamster kidney (BHK) cells, NSO- and SP2/0-mouse myeloma cells.

In an embodiment, the antibodies according to the invention are produced or expressed in mammal cells, preferably wild-type mammal cells, preferably of rodent origin, especially CHO cells.

Modifications and changes may be made in the structure of an antibody of the present invention and still obtain a molecule having like characteristics. For example, certain amino acids can be substituted for other amino acids in a sequence without appreciable loss of activity. Because it is the interactive capacity and nature of an antibody that defines that antibody's biological functional activity, certain amino acid sequence substitutions can be made in an antibody sequence (or, of course, its underlying DNA coding sequence) and nevertheless obtain an antibody with like properties.

In making such changes, the hydropathic index of amino acids can be considered. The importance of the hydropathic amino acid index in conferring interactive biologic function on an antibody is generally understood in the art. It is known that certain amino acids can be substituted for other amino acids having a similar hydropathic index or score and still result in an antibody with similar biological activity. Each amino acid has been assigned a hydropathic index on the basis of its hydrophobicity and charge characteristics.

It is believed that the relative hydropathic character of the amino acid determines the secondary structure of the resultant antibody, which in turn defines the interaction of the antibody with other molecules, for example, enzymes, substrates, receptors, antibodies, antigens, and the like. It is known in the art that an amino acid may be substituted by another amino acid having a similar hydropathic index and still obtain a biologically functionally equivalent polypeptide. In such changes, the substitution of amino acids whose hydropathic indices are within +2 is preferred, those which are within +1 are particularly preferred, and those within +0.5 are even more particularly preferred.

Substitution of like amino acids can also be made on the basis of hydrophilicity, particularly where the biologically functionally equivalent peptide or polypeptide thereby created is intended for use in immunological embodiments. U.S. Patent 4,554,101, incorporated herein by reference or to which the person skilled in the art : may refer, states that the greatest local average hydrophilicity of a polypeptide, as governed by the hydrophilicity of its adjacent amino acids, correlate with its immunogenicity and antigenicity, *i.e.* with a biological property of the polypeptide.

As detailed in U.S. Patent 4,554,101, the following hydrophilicity values have been assigned to amino acid residues: arginine (+3.0); lysine (+3.0); aspartate (+3.0 ±1); glutamate (+3.0 ±1); serine (+0.3); asparagine (+0.2); glutamine (+0.2); glycine (0); proline (-0.5 ±1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent, polypeptide. In such changes, the substitution of amino acids whose hydrophilicity values are within +2 is preferred, those which are within +1 are particularly preferred, and those within +0.5 are even more particularly preferred.

As outlined above, amino acid substitutions are generally therefore based on the relative similarity of the amino acid side-chain substituents, for example, their hydrophobicity, hydrophilicity, charge, size, and the like.

Amino acid substitution may be chosen or selected differently. Possible substitutions have been documented in WO99/51642, WO2007024249 and WO2007106707.

By definition, the CDRs of the invention include variant CDRs, by deletion, substitution or addition of one or more amino acid(s), which variant keeps the specificity of the original CDR and the whole VH + VL or antibody, substantially keeps the antigen-binding of the parental Fab or antibody.

Engineered antibodies of the invention include those in which modifications have been made to framework residues within VH and/or VL, e.g. to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention. Another type of framework modification involves mutating one or more residues within the framework region, or even within one or more CDR regions, to remove T cell -epitopes to thereby reduce the potential immunogenicity of the antibody. This approach is also referred to as "deimmunization" and is described in further detail in U.S. Patent Publication No. 20030153043 by Carr et al.

### Nucleic acids and vectors

The isolated nucleic acid sequences disclosed and provided herein are also object of the invention.

Thus the invention also relates to an isolated nucleotide sequence selected from the group consisting of the nucleotide sequences SEQ ID NO: 14, 16, 18, 20, 22, 24, 26, 28, 30, 32, and more particularly combinations or sets of two nucleotide sequences separated or linked together: SEQ ID NO: 14 and 16, 18 and 20, 22 and 24, 26 and 28, 30 and 32.

As mentioned above, methods for producing the antibodies are known from the person skilled in the art. The mammal cells, preferably rodent cells such as CHO cells, preferably wild-type cells are transfected with one or several expression vectors. Preferably, the cells are co-transfected with an expression vector for light chain and with an expression vector for heavy chain. Cell transfection is also known from the person skilled in the art. As transfection that may be performed, one may mention without limitation standard transfection procedures, well-known from the man skilled in the art, such as calcium phosphate precipitation, DEAE-Dextran mediated transfection, electroporation, magnetofection, nucleofection (AMAXA Gmbh, GE), liposome-mediated transfection (using dreamfect®, lipofectin® or lipofectamine® technology for example) or microinjection.

Expression vectors are known. As vectors that may be used, one may mention without limitation: pcDNA3.3, pOptiVEC, pFUSE, pMCMVHE, pMONO, pSPORT1, pcDV1, pcDNA3, pcDNA1, pRc/CMV, pSEC. One may use a single expression vector or several expression vectors expressing different parts of the antibody.

The invention also relates to an expression vector encoding a Heavy Chain of an anti-CD117 antibody, an expression vector encoding a Light Chain of an anti-CD117 antibody, or an expression vector encoding a Heavy Chain and a Light Chain of an anti-CD117 antibody.

Another object of the invention is a host cell containing a vector or a set of vectors of the invention. The host cell may be a mammal cell, preferably a rodent cell, more preferably CHO cell. Still more preferably, the host cell may be a wild-type mammal cell, preferably a wild-type rodent cell, most preferably a wild-type CHO cell.

The person skilled in the art fully owns the methods to generate the antibodies according to the invention using such a vector or vectors and cells such as CHO cells.

### BRIEF DESCRIPTION OF THE DRAWINGS

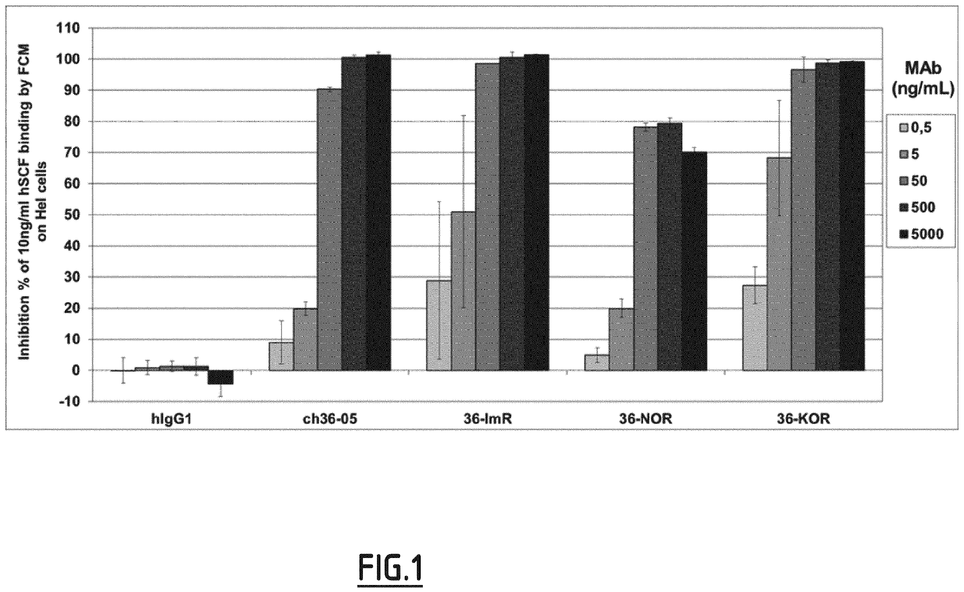

FIGURE 1 is a bar diagram showing percent (%) of the inhibition of SCF binding (100 ng/mL, FACS analysis) in the presence of antibody tested at different concentrations using HEL cells (5.10⁵ cells/mL), (mean +/- SD, n=2), in the presence of 10 ng/ml of SCF.

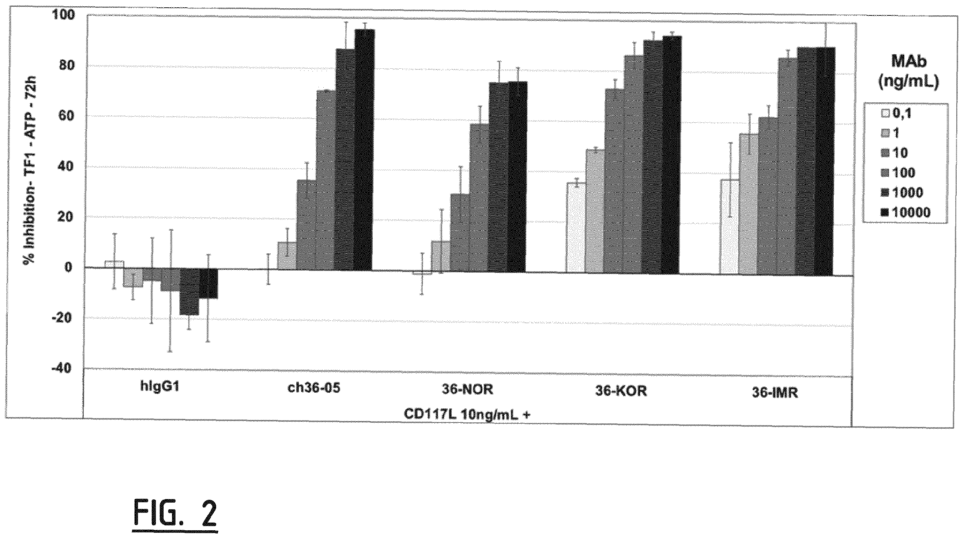

FIGURE 2 is a bar diagram showing percent (%) of the cell proliferation inhibition (ATP bioluminescent bioassay, 72 hours) of antibody tested at different concentrations using HEL cells (5.10⁴ cells/mL), (mean +/- SD, n=2) in the presence of 10 ng/ml of SCF.

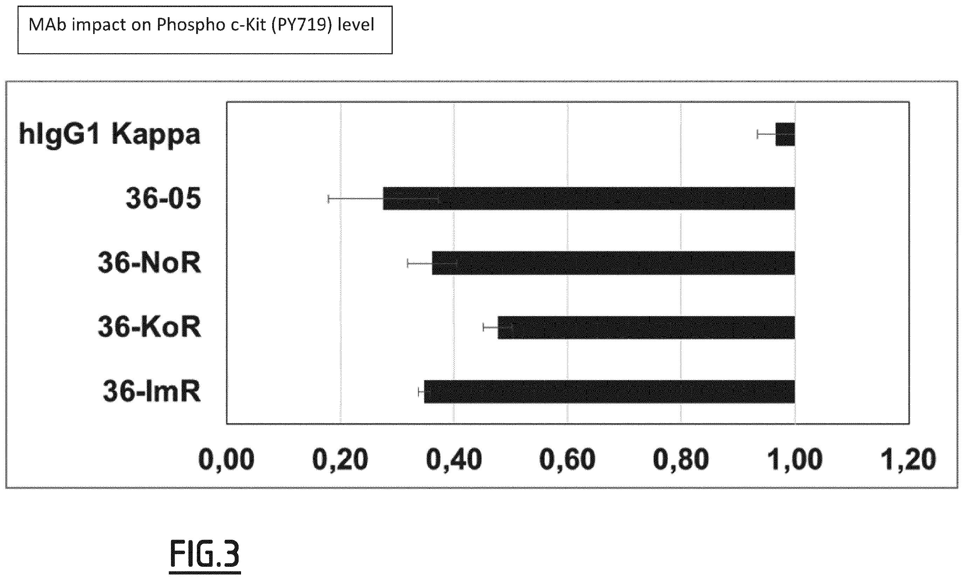

FIGURE 3 is bar diagram showing western blot data related to the level of native c-kit detected by the polyclonal detecting no Phospho-CD117/c-Kit (PY719) following TF1 cell incubation in the presence of SCF with different anti-CD117 monoclonal antibodies.

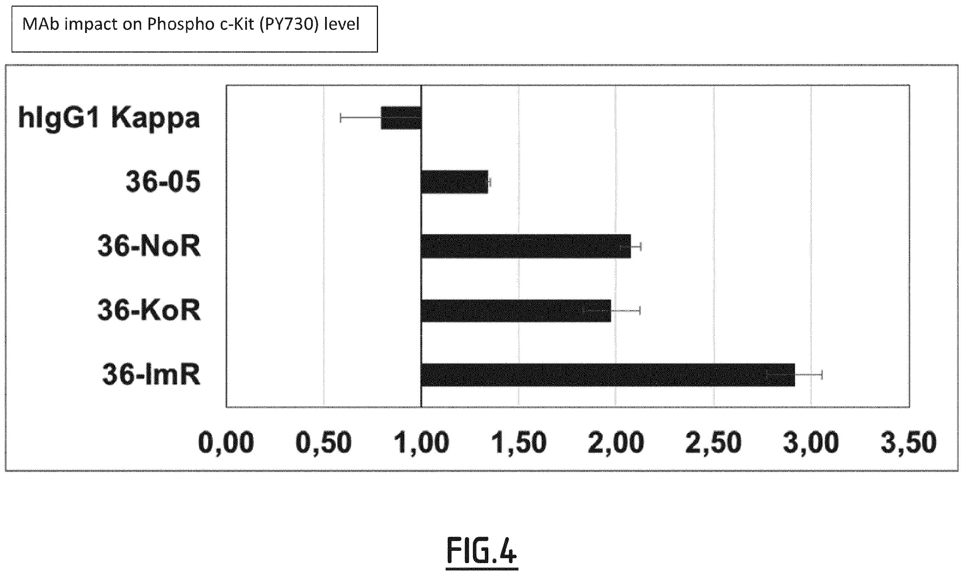

FIGURE 4 is bar diagram showing western blot data related to the level of native c-kit detected by the polyclonal detecting Phospho-CD117/c-Kit (PY730) following TF1 cell incubation in the presence of SCF with different anti-CD117 monoclonal antibodies.
FIGURE 5 Amino acid and nucleic acid sequence for murine or chimeric VH R036-05 with description of the FR1, CDR1, FR2, CDR2, FR3, CDR3 defined according IMGT.
FIGURE 6: Amino acid and nucleic acid sequence for murine or chimeric VL R036-05 with description of the FR1, CDR1, FR2, CDR2, FR3, CDR3, defined according IMGT.

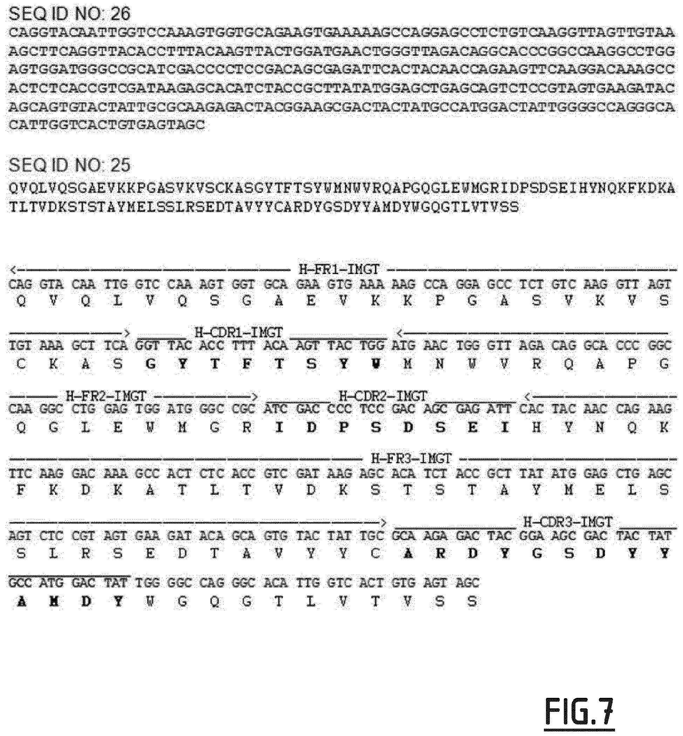

FIGURE 7: Amino acid and nucleic acid sequence for humanized VH 36.5Hz27 with description of the FR1, CDR1, FR2, CDR2, FR3, CDR3, defined according IMGT.
FIGURE 8: Amino acid and nucleic acid sequence for humanized VL Hz 36.5Hz31 with description of the FR1, CDR1, FR2, CDR2, FR3, CDR3, defined according IMGT.

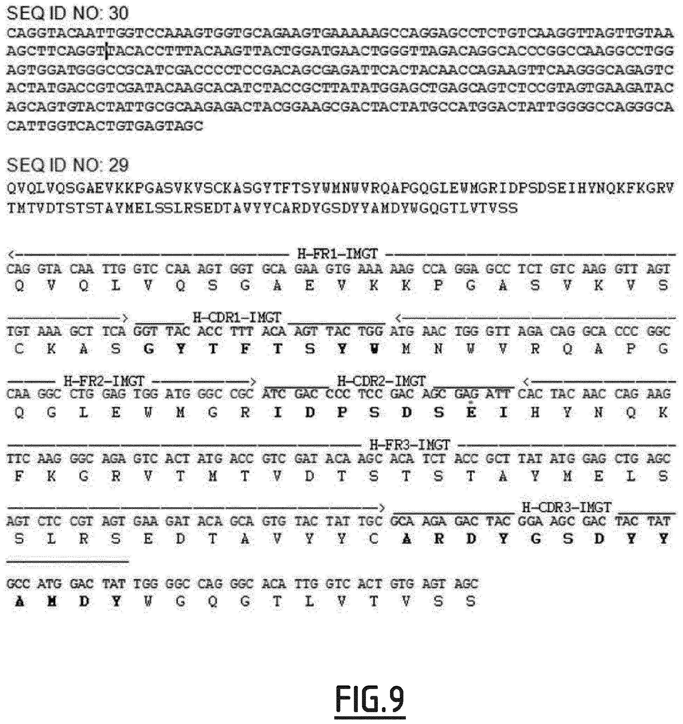

FIGURE 9: Amino acid and nucleic acid sequence for humanized VH 36.5Hz30 with description of the FR1, CDR1, FR2, CDR2, FR3, CDR3, defined according IMGT.

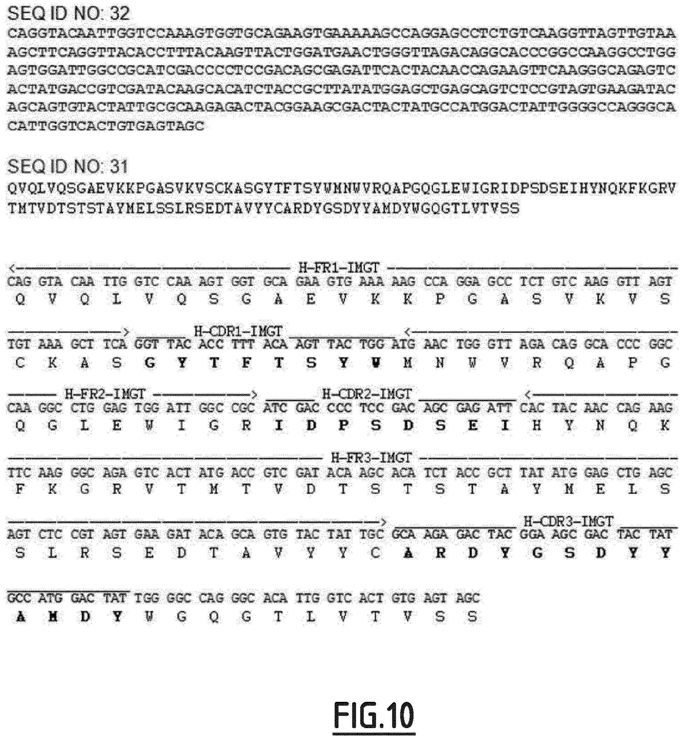

FIGURE 10: Amino acid and nucleic acid sequence for humanized VH Hz 36.5Hz31 with description of the FR1, CDR1, FR2, CDR2, FR3, CDR3, defining according IMGT.

### EXAMPLES

The following examples are offered to illustrate, but not to limit the claimed invention.

### EXAMPLE 1: Preparation of murine anti-CD117 antibody

This example illustrates the preparation of hybridoma cell lines secreting anti-CD117 antibodies.

The murine monoclonal antibodies specific for CD117 were produced using standard hybridoma techniques (Zola et al., Aust J. Exp Biol Med Sci. 1981; 59:303-6). After hybridoma cloning, one murine Mab was obtained called 36-05 (also named B-K15, Diaclone, France). The clone was injected into the peritoneum of nude mice. Protein A chromatography from murine ascitic fluid. The murine ascitic fluid is adjusted at pH 8.3 with the equilibration buffer 0.1 M Tris and 1.5 M Sulfate Ammonium and then loaded onto the rProtein A Sepharose Fast Flow column (GE Healthcare, Saint Cyr au Mont d'or, France). The non-binding proteins are flowed through and removed by several washings with equilibration buffer. The anti-CD117 monoclonal antibody (Mab anti-CD117) is eluted off the Protein A column using the elution buffer 0.1 M Citrate Sodium at pH 3.5. After concentration, the PBS solution containing IgG was filtered and the Mab concentration was determined at 280 nm.

A chimeric version of this 36-05 was produced as disclosed herein to get the ch36-05 monoclonal antibody made of Heavy chain of SEQ ID NO: 21 and of Light chain of SEQ ID NO: 23.

### EXAMPLE 2: Preparation of comparative anti-CD117 monoclonal antibodies

Comparative monoclonal antibodies were prepared on the following basis:
- 36-NOR, based on sequences H-NEH085 and L-NEH0855 disclosed in WO02016020791 ;
- 36-KOR, based on sequences H-37M and L-37M disclosed in WO2015050959; and WO2015112822;
- 36-IMR, based on sequences HCVR and LCVR disclosed in US 8,552,157.

### EXAMPLE 3: Cell culture

Various tumour-derived cell lines are among the target cells that may be stained with MAb anti-CD117, in such assay procedures.

Cell lines. The established human erythroleukemia cells HEL (available from DSMZ were grown in RPMI-1640 Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin -streptomycin (Sigma, St Quentin Fallavier, France). The established human erythroleukemia cells TF1 (available from ECACC) was grown in RPMI-1640 Medium (Sigma, St Quentin Fallavier, France) supplemented with 10% heat-inactivated fetal bovine serum (FBS) (Sigma, St Quentin Fallavier, France), 4 nM L-glutamine (Sigma, St Quentin Fallavier, France) and 100 U/mL, 100 µg/mL penicillin -streptomycin (Sigma, St Quentin Fallavier, France), 1% sodium pyruvate (Sigma, St Quentin Fallavier, France) and 5ng/mL GM-CSF (R&D systems, Lille, France)

### EXAMPLE 4: MAb competition by Flow cytometry

Flow cytometry experiments for MAb competition binding. Briefly, 2.10⁵ cells HEL or TF1 per 96 wells are incubated with or without different concentration of unconjugated antibody and incubated at 4°C for 30 min. Then different concentrations of biotinylated murine anti-CD117 antibody as a reference were added and incubated at 4°C for 30 min. Only data obtained with 1 µg/mL of biotinylated antibody is shown. Unbound antibody is washed away with PBS (Invitrogen, Villebon sur Yvette, France) supplemented by 1% Bovine Serum Albumin (Sigma, St Quentin Fallavier, France). Subsequently, cells are centrifuged (5 min at 400 g) and bound antibody is detected with Phycoerythrin conjugated Streptavidin (Interchim, Montluçon, France) at 4°C for 30 min. Detection reagent is washed away and cells are centrifuged (5 min at 400 g) and resuspended in 300 µL PBS. Bound detection antibody is quantified on a FACScan or a FACScalibur (BD Biosciences, Rungis, France), (FL2 channel, at least 2000 events per acquisition.

Table 3 shows the results of the level of MAb competition (%) resulting of the inhibition of biotinylaled MAb binding (1 µg/mL, FACS analysis) in the presence of other unconjugated antibody (5 g/mL) using the HEL cells (1 .10⁶ cells/mL), (mean +/- SD, n=2).

| | Biotinylated Mab |
|---|---|
| Codification | R036-05 |
| hlgG1 | - |
| ch36-05 | +++ |
| 36-NOR | - |
| 36-KOR | - |
| 36-IMR | +++ |

| | |
|---|---|
| Legend: % of inhibition of mean fluorescence intensity of cell labeling with biotinylated MAb in the presence of unconjugated Mab: - : -10%; + : 10-40%; ++ : 50%; +++: 90%) | |

For example the unconjugated ch36-05 is not in competition with the biotinylated 36-NOR or 36-KOR. By contrast, unconjugated ch36-05 is in competition with the biotinylated 36-IMR. Therefore, the epitope ch36-05 is not common or adjacent with the epitopes 36-NOR or 36-KOR.

### EXAMPLE 5: Antibody potency on SCF binding

Flow cytometry experiments of MAb impact on human SCF binding. HEL cell lines were seeded at a density of 1.10⁵ per 96-wells. Cells were incubated for 30 min at 4°C with or without MAb anti-CD117 tested at 5 µg/mL then diluted at 1/10. Unbound antibodies were washed away with PBS (Invitrogen, Villebon sur Yvette, France) supplemented by 1% Bovine Serum Albumin (Sigma, St Quentin Fallavier, France). Subsequently, cells are incubated with the human SCF (10 ng/mL), for 30 min at 4°C. Unbound antibodies were washed away with PBS (Invitrogen, Villebon sur Yvette, France) supplemented by 1% Bovine Serum Albumin (Sigma, St Quentin Fallavier, France). The bound human SCF is detected with biotinylated conjugated goat anti-human SCF (R&D Systems, Lille, France). After washings, Phycoerthrin conjugated Streptavidin (Interchim, Montluçon, France) was added at 4°C for 30 min. Detection reagent is washed away and cells are centrifuged (5 min at 400 g) and resuspended in 300 µL PBS. Bound detection antibody is quantified on a FACSCAN (BD Biosciences, Rungis, France), (FL2 channel, 2 000 events per acquisition). During the experiment, the respective isotype controls are included to exclude any unspecific binding events.

Results of experiments to determine the antagonist activity on SCF binding of anti-CD117 antibodies are shown in FIGURE 1. All of the anti-CD117 MAbs inhibit human SCF binding on HEL CD117 positive cells.

### EXAMPLE 6: Antibody potency on SCF induced cell proliferation

Biochemical reagents. Biochemical reagents used for the cell proliferation related studies were: recombinant human SCF (R&D Systems, Lille, France) and Cell Titer GLo-ATP (Promega, Charbonnières-les-bains, France).

Cell viability analysis following ATP level determination. The CellTiter-Glo® Luminescent Cell Viability Assay (Promega, Charbonnières les Bains, France) was used to determine the number of viable cells in culture based on quantification of the ATP present, an indicator of metabolically active cells. Detection is based on using the luciferase reaction to measure the amount of ATP from viable cells. Within minutes after a loss of membrane integrity, cells lose the ability to synthesize ATP, and endogenous ATPases destroy any remaining ATP; thus the levels of ATP fall precipitously. Human erythroleukemia cell line TF1 show complete growth dependency on GM-CSF. Cell cultures are incubated for 24 hours before the test with culture medium without GM-CSF and only 1% FCS. Cell cultures (5.10⁴ cells/mL) are incubated for 72 at 37°C hours with different concentration of interest MAbs and controls. The human SCF concentration was used at 10 ng/mL. The CellTiter-Glo® reagent was added directly to cells in culture at a ratio of 50µL of reagent to 200µL of culture medium. The assay plates are incubated at room temperature for 10 min and the bioluminescent signal is recorded using a standard multiwell fluorometer Mithras LB940, (Berthold, Thoiry, France).

Results of experiments to determine the antagonist activity of anti-CD117 antibodies on SCF induced myeloid cell proliferation are shown in FIGURE 2. All of the anti-CD117 MAbs inhibit human SCF induced TF1 cell proliferation.

### EXAMPLE 7: Antibody potency on c-Kit phosphorylation

Activation of CD117 occurs when an SCF dimer binds to its extracellular domain. CD117 phosphorylation triggers several signal transduction pathways, including JAK/STAT, RAS/MAP kinase pathway, PI3 kinase, PLCγ pathway, and SRC pathway. TF1 cells were serum-starved 24 hours at a density of 0,5.10⁶ cells/ml and the following day treated in presence of anti-CD117 MAbs, benchmark MAbs or control IgG at 10 µg/ml for 30 minutes at 37 °C. Human SCF (R&D Systems, Lille, France) stimulation at 10 ng/mL was performed for 10 min at 37 °C. Cells were rinsed in PBS1X (Life Technologies, Courtaboeuf, France) and lysates prepared in cell lysis buffer (Life Technologies, Courtaboeuf, France) containing Protease 1X (Sigma, St Quentin Fallavier, France) and Phosphatase Inhibitor cocktails 1X (Life Technologies, Courtaboeuf, France). Samples were sonicated on ice 10 times (amplitude value 70 %) for 10 seconds on / 50 seconds off. Lysate analysis was performed on NuPage 4-12% Bis-Tris Midigels (Life Technologies, Courtaboeuf, France) and protein transferred onto Immuno-Blot PVDF membrane (Biorad, Oxfordshire, United Kingdom). Blots were incubated with primary antibody dilutions: 1:500 Phospho-CD117/c-Kit (Y719) antibody (Life Technologies, Courtaboeuf, France), 1:500 Phospho-CD117/c-Kit (Y730) antibody (Life Technologies, Courtaboeuf, France), 1:2 000 CD117/c-Kit antibody (R&D Systems, Lille, France) and 1:10 000 Histone H3 antibody (Abcam, Cambridge, United Kingdom). Secondary antibody-HRP conjugates were used at 1/10000 (Biorad, Oxfordshire, United Kingdom) and at 1/500 (R&D Systems, Lille, France). Enhanced chemiluminescence (ECL) reagents (GE Healthcare, Saint Cyr au Mont d'or, France) were used to detect protein band signal. Enhanced chemiluminescence (ECL) reagents (Amersham; RPN2235) were used to detect protein band signal. All blot images were captured with an PXi4 imaging system (Syngene). Band density analysis were performed using Gene Tools software from Syngene. Band density value was attributed (#100) to Ctrl Human IgG1 band. Band density values were calculated for each band according to this reference value, for all PAbs tested : Anti-Histone H3 (Loading control - PAbO), Anti-c-kit (PAb1) and Anti-Phospho-c-kit (PY719) / PAb4 or PY730 / PAb6). Band density Ratios [Anti-(Phospho)-c-kit / Anti-Histone H3] were calculated for each condition tested. For a ratio ≈ 1, its means no variation for (Phospho)-c-kit detection level. For a ratio > 1, its means a up-regulation (Phospho)-c-kit detection and with a ratio < 1, its means a down-modulation of (Phospho)-c-kit detection level by Western Blot compared with Histone H3.
Results of experiments to determine the antagonist activity of anti-CD117 antibodies on CD117 activation on TF1 cells are shown in FIGURE 3. Western-blot analysis showed that all tested anti-CD117 antibodies tested down-modulate stem-cell factor induced PY719 phosphorylated CD117 (Figure 3). By contrast all of anti-CD117 antibodies excepted antibody R036-05 significantly up-modulate stem-cell factor induced PY730 phosphorylated CD117 (Figure 4). Thus the R036-05 antibody according to the invention selectively induces a restricted transduction pathway on transphosphorylation of tyrosine c-kit residues. The major substrates for activated KIT are specific tyrosine residues contained within the cytosolic domain of KIT. Therefore restricted Pi CD117 will generate subsequent events differing compared to the other tested anti-CD117 antibodies. (gene transcription, cell adhesion, cell proliferation, Phospholipase Cy (PLCy), phosphoinositol-3-kinase (PI3K), protein tyrosine phosphatase 1C (PTP1C), mitogen-activate kinase kinase (MAPKK).

### EXAMPLE 8: Peptide mapping by ELISA

ELISA experiments were performed by using a panel of 52 peptides covering the extracellular domain of CD117 antigen. Each BSA conjugated peptide vas coated on 96 wells ELISA plates, (from 50 to 100 ng/mL) The MAb panel was tested from 10 to 0.01 µg/mL and revealed by using a goat polyclonal anti specie (mouse or human) IgG1 Horse Radish Peroxydase (HRP) conjugated (AbD Serotec, Colmar, France).

Among the panel of coated BSA peptides (52 peptides) related to the extracellular region of CD117, some of them are detected with the 36-NOR, 36-KOR and 36-ImR. However, R036-05 did not detect them, revealing that the R036-05 antibody is recognizes a conformational CD117 epitope (data not shown).

### EXAMPLE 9: Preparation of chimeric monoclonal antibody directed against CD117

DNA encoding the monoclonal antibodies is readily isolated and sequenced using conventional procedures (e.g. by using oligonucleotide probes that are capable of binding specifically to genes encoding the heavy and light chains of murine antibodies). Conversion of murine MAb to native chimeric MAb:
The cDNA corresponding to the variable region of the hybridoma was obtained using two approaches. The first approach consisted in the use in PCR of the degenerate N-term amino acid related primer set generated from the N-Terminal sequencing. The second approach consisted in the use in PCR of degenerate primer set generated by IMGT® primer database and specific primers previously described *(*Essono et al., J Immunol Methods. 2003; 203: 279:25-66*,* Wang et al., Mol Immunol. 1991; 28:1387-97*).* The sequence of N-terminal variable region was determined by Edman degradation. Total RNA extraction was carried out using the Tri Reagent kit according to the protocol described by the supplier Sigma. The amplified VL and VH fragments were cloned into the TOPO-TA cloning vector (Invitrogen) for sequence analyses by the dideoxytermination method *(*Sanger et al., Nature. 1977; 265:687-95*).* Then antibody variant constructs were amplified by PCR and cloned into the expression vector.

Positions were numbered according to IMGT and to Kabat index (Identical V region amino acid sequences and segments of sequences in antibodies of different specificities). Relative contributions of VH and VL genes, minigenes, and complementarity-determining regions to binding of antibody-combining sites were analyzed (Kabat et al., NIH Publ. 1991; No. 91-3242, Vol. 1, 647-669).

### EXAMPLE 10: Preparation of humanized monoclonal antibodies

Antibody CDR and FR regions have been determined according to various numbering approaches such as IMGT (ImMunoGeneTics Information SystemR http://imgt.cines.fr), Kabat or Common Numbering System. However, IMGT determined CDRs for a given antibody are not necessarily identical to the CDRs defined by the other numbering systems. The CDRs and framework regions (FR) have been identified by the inventor thanks to IMGT numbering systems. (ImMunoGeneTics Information SystemR http://imgt.cines.fr). For Chotia, reference can be done to Antibodies - www.bioinf.org.uk.

Conversion of chimeric MAb to Humanized MAb:
Humanized CD117 antibody Heavy (H) and Light (L) chain were generated using CDR-grafting by the PCR method. In order to generate a humanized antibody in which the CDRs of a mouse monoclonal antibody are grafted onto a human antibody, there is preferably a high homology between the variable region of a mouse monoclonal antibody and the variable region of a human antibody. Thus, the H chain and L chain V regions of a mouse anti-human CD117 monoclonal antibody are compared to the V region of all known human antibodies using the software IMGT/DomainGapAlign. When a mouse antibody is humanized by a conventional technology, the amino acid sequence of some of the V region FRs of a mouse antibody supporting the CDR may be grafted onto the FR of a human V region, as desired. Then the sequence of the Humanized variable region of is determined. The variables regions of H and L were amplified by PCR and cloned into the expression vector p3U containing the human IgG1 constant region. Three humanized antibodies were selected sharing a common light chain, but with three distinct heavy chains as the antibody 36-5 Hz114 related to Heavy chain SEQ ID NO: 25 and Light chain SEQ ID NO: 27, the antibody 36-5 Hz121 related to Heavy chain SEQ ID NO: 29 and Light chain SEQ ID NO: 27; and the antibody 36-5 Hz128 Heavy chain SEQ ID NO: 31 and Light chain SEQ ID NO: 27.

By flow cytometry, the humanized mAb staining was analyzed on HEL CD117 positive cell line. As shown in table 4, similar mAb cell staining (percentage of labelled cells / mean fluorescence Intensity) was observed whatever the mAb design (chimeric versus humanized mAb). No staining was observed on the cell line CEM (CD117 negative, (data not shown).

**Table 4 : Comparative mAb analysis by flow cytometry of HEL cellular staining according the mA design.**

| | | µl/2.10⁵cells | | | |
|---|---|---|---|---|---|
| | | 100 | 10 | 1 | 0,1 |
| **mAb design** | **ref** | **% labelled HEL cells** / **MFI** | | | |
| chimeric mAb | chR036-05 | 80/251 | 81/262 | 80/259 | 43/259 |
| humanized mAb | 36-5 Hz114 | 74/242 | 70/136 | 66/228 | 64/225 |
| humanized mAb | 36-5 Hz121 | 73/241 | 70/236 | 67/228 | 59/210 |
| humanized mAb | 36-5 Hz128 | 94/291 | 77/250 | 72/240 | 61/214 |

## Claims

1. An antibody, preferably monoclonal antibody, or a fragment thereof, said antibody or fragment thereof specifically binding to CD117 and comprising
- (1) a VH chain comprising the following three CDRs: a H-CDR1 of sequence SEQ ID NO: 1, a H-CDR2 of sequence SEQ ID NO: 2, a H-CDR3 of sequence SEQ ID NO: 3; and a VL chain comprising the following three CDRs: a L-CDR1 of sequence SEQ ID NO: 4, a L-CDR2 of sequence STS, a L-CDR3 of sequence SEQ ID NO: 5;
- (2) a VH chain comprising the following three CDRs: a H-CDR1 of sequence SEQ ID NO: 33, a H-CDR2 of sequence SEQ ID NO: 2, a H-CDR3 of sequence SEQ ID NO: 3; and a VL chain comprising the following three CDRs: a L-CDR1 of sequence SEQ ID NO: 36, a L-CDR2 of sequence STS, a L-CDR3 of sequence SEQ ID NO: 5;
- (3) a VH chain comprising the following three CDRs: a H-CDR1 of sequence SEQ ID NO: 6, a H-CDR2 of sequence SEQ ID NO: 7, a H-CDR3 of sequence SEQ ID NO: 8; and a VL chain comprising the following three CDRs: a L-CDR1 of sequence SEQ ID NO: 9, a L-CDR2 of sequence SEQ ID NO: 10, a L-CDR3 of sequence SEQ ID NO: 5;
- (4) a VH chain comprising the following three CDRs: a H-CDR1 of sequence SEQ ID NO: 34, a H-CDR2 of sequence SEQ ID NO: 7, a H-CDR3 of sequence SEQ ID NO: 8; and a VL chain comprising the following three CDRs: a L-CDR1 of sequence SEQ ID NO: 37, a L-CDR2 of sequence SEQ ID NO: 10, a L-CDR3 of sequence SEQ ID NO: 5;
- (5) a VH chain comprising the following three CDRs: a H-CDR1 of sequence SEQ ID NO: 11, a H-CDR2 of sequence SEQ ID NO: 12, a H-CDR3 of sequence SEQ ID NO: 8; and a VL chain comprising the following three CDRs: a L-CDR1 of sequence SEQ ID NO: 9, a L-CDR2 of sequence SEQ ID NO: 10, a L-CDR3 of sequence SEQ ID NO: 5;
- (6) a VH chain comprising the following three CDRs: a H-CDR1 of sequence SEQ ID NO: 35, a H-CDR2 of sequence SEQ ID NO: 12, a H-CDR3 of sequence SEQ ID NO: 8; and a VL chain comprising the following three CDRs: a L-CDR1 of sequence SEQ ID NO: 37, a L-CDR2 of sequence SEQ ID NO: 10, a L-CDR3 of sequence SEQ ID NO: 5;
- (7) a VH chain comprising the following three CDRs: a H-CDR1 of sequence RYW, a H-CDR2 of sequence SEQ ID NO: 2, a H-CDR3 of sequence SEQ ID NO: 8; and a VL chain comprising the following three CDRs: a L-CDR1 of sequence SEQ ID NO: 4, a L-CDR2 of sequence STS, a L-CDR3 of sequence SEQ ID NO: 5; or
- (8) a VH chain comprising the following three CDRs: a H-CDR1 of sequence SYW, a H-CDR2 of sequence SEQ ID NO: 2, a H-CDR3 of sequence SEQ ID NO: 8; and a VL chain comprising the following three CDRs: a L-CDR1 of sequence SEQ ID NO: 36, a L-CDR2 of sequence STS, a L-CDR3 of sequence SEQ ID NO: 5,
for use in treating cancer expressing CD117.

2. The antibody or fragment thereof for the use of claim 1, comprising a VH region of sequence SEQ ID NO: 13 and a VL region of sequence SEQ ID NO: 15.

3. The antibody or fragment thereof for the use of claim 1, comprising
- (1) a Heavy Chain comprising a VH region comprising the following three CDRs: a H-CDR1 of sequence SEQ ID NO: 1, a H-CDR2 of sequence SEQ ID NO: 2, a H-CDR3 of sequence SEQ ID NO: 3, and a human Fc region, preferably an IgG1 human Fc region; and a Light Chain comprising the following three CDRs: a VL region comprising a L-CDR1 of sequence SEQ ID NO: 4, a L-CDR2 of sequence STS, a L-CDR3 of sequence SEQ ID NO: 5, and a human Kappa region;
- (2) a Heavy Chain comprising a VH region comprising the following three CDRs: a H-CDR1 of sequence SEQ ID NO: 33, a H-CDR2 of sequence SEQ ID NO: 2, a H-CDR3 of sequence SEQ ID NO: 3, and a human Fc region, preferably an IgG1 human Fc region; and a Light Chain comprising the following three CDRs: a VL region comprising a L-CDR1 of sequence SEQ ID NO: 36, a L-CDR2 of sequence STS, a L-CDR3 of sequence SEQ ID NO: 5, and a human Kappa region;
- (3) a Heavy Chain comprising a VH region comprising the following three CDRs: a H-CDR1 of sequence SEQ ID NO: 6, a H-CDR2 of sequence SEQ ID NO: 7, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 9, a L-CDR2 of sequence SEQ ID NO: 10, a L-CDR3 of sequence SEQ ID NO: 5, and a human Kappa region;
- (4) a Heavy Chain comprising a VH region comprising the following three CDRs: a H-CDR1 of sequence SEQ ID NO: 34, a H-CDR2 of sequence SEQ ID NO: 7, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 37, a L-CDR2 of sequence SEQ ID NO: 10, a L-CDR3 of sequence SEQ ID NO: 5, and a human Kappa region;
- (5) a Heavy Chain comprising a VH region comprising the following three CDRs: a H-CDR1 of sequence SEQ ID NO: 11, a H-CDR2 of sequence SEQ ID NO: 12, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 9, a L-CDR2 of sequence SEQ ID NO: 10, a L-CDR3 of sequence SEQ ID NO: 5, and a human Kappa region;
- (6) a Heavy Chain comprising a VH region comprising the following three CDRs: a H-CDR1 of sequence SEQ ID NO: 35, a H-CDR2 of sequence SEQ ID NO: 12, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 37, a L-CDR2 of sequence SEQ ID NO: 10, a L-CDR3 of sequence SEQ ID NO: 5, and a human Kappa region;
- (7) a Heavy Chain comprising a VH region comprising the following three CDRs: a H-CDR1 of sequence RYW, a H-CDR2 of sequence SEQ ID NO: 2, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 4, a L-CDR2 of sequence STS, a L-CDR3 of sequence SEQ ID NO: 5, and a human Kappa region; or
- (8) a Heavy Chain comprising a VH region comprising the following three CDRs: a H-CDR1 of sequence SYW, a H-CDR2 of sequence SEQ ID NO: 2, a H-CDR3 of sequence SEQ ID NO: 8, and a human Fc region, preferably an IgG1 human Fc region; and a Light Chain comprising the following three CDRs: a VL region comprising a L-CDR1 of sequence SEQ ID NO: 36, a L-CDR2 of sequence STS, a L-CDR3 of sequence SEQ ID NO: 5, and a human Kappa region.

4. The antibody or fragment thereof for the use of claim 1, comprising a chimeric Heavy Chain of sequence SEQ ID NO: 21; a chimeric Light Chain of sequence SEQ ID NO: 23; or a chimeric Heavy Chain of sequence SEQ ID NO: 21 and a chimeric Light Chain of sequence SEQ ID NO: 23.

5. The antibody or fragment thereof for the use of claim 1, which is a humanized antibody or fragment thereof, specifically binding to CD117.

6. The antibody or fragment thereof for the use of claim 1 or 5, comprising
(i) a VH of sequence SEQ ID NO: 25 and a VL of sequence SEQ ID NO: 27;
(ii) a VH of sequence SEQ ID NO: 29 and a VL of sequence SEQ ID NO: 27;
(iii) a VH of sequence SEQ ID NO: 31 and a VL of sequence SEQ ID NO: 27..

7. The antibody or fragment thereof for the use of claim 1 or 5, comprising:
(i) a Heavy Chain comprising a VH region of sequence SEQ ID NO: 25 and a human Fc region, preferably an IgG1 human Fc region; and a Light Chain comprising a VL region of sequence SEQ ID NO: 27 and a human Kappa region;
(ii) a Heavy Chain comprising a VH region of sequence SEQ ID NO: 29 and a human Fc region, preferably an IgG1 human Fc region; and a Light Chain comprising a VL region of sequence SEQ ID NO: 27 and a human Kappa region;
(iii) a Heavy Chain comprising a VH region of sequence SEQ ID NO: 31 and a human Fc region, preferably an IgG1 human Fc region; and a Light Chain comprising a VL region of sequence SEQ ID NO: 27 and a human Kappa region.

8. The antibody for the use according to any one of claims 1 to 7, wherein the antibody is a monoclonal antibody.

9. The fragment of antibody for the use according to any one of claims 1 to 7, wherein the fragment is a F(ab')2, a Fab, a Fab', a dsFv, a (dsFv)2, a scFv, a sc(Fv)2, a diabody, a triabody, a minibody, a nanobody, a scFv-Fc, and a multimeric scFv.

10. A humanized monoclonal antibody or a fragment thereof, said humanized antibody or fragment thereof specifically binding to CD117 and comprising
(i) a VH of sequence SEQ ID NO: 25 and a VL of sequence SEQ ID NO: 27;
(ii) a VH of sequence SEQ ID NO: 29 and a VL of sequence SEQ ID NO: 27;
(iii) a VH of sequence SEQ ID NO: 31 and a VL of sequence SEQ ID NO: 27.

11. The humanized monoclonal antibody of claim 10, further comprising a human Fc region, preferably an IgG1 human Fc region, and/or a human Kappa region.

12. The humanized monoclonal antibody of claim 10, comprising:
(i) a Heavy Chain comprising a VH region of sequence SEQ ID NO: 25 and a human Fc region, preferably an IgG1 human Fc region; and a Light Chain comprising a VL region of sequence SEQ ID NO: 27 and a human Kappa region;
(ii) a Heavy Chain comprising a VH region of sequence SEQ ID NO: 29 and a human Fc region, preferably an IgG1 human Fc region; and a Light Chain comprising a VL region of sequence SEQ ID NO: 27 and a human Kappa region;
(iii) a Heavy Chain comprising a VH region of sequence SEQ ID NO: 31 and a human Fc region, preferably an IgG1 human Fc region; and a Light Chain comprising a VL region of sequence SEQ ID NO: 27 and a human Kappa region.

13. The humanized monoclonal antibody or fragment thereof of any one of claims 10 to 12, as a medicament.

14. An anticancerous composition comprising an antibody or fragment thereof according to any one of claims 1 to 12, and a pharmaceutically acceptable vehicle, carrier or excipient.

15. The composition according to claim 14, for use as a medicament against a cancer expressing CD117.


SEQUENCE LISTING

<110>  INTERNATIONAL DRUG DEVELOPMENT BIOTECH

<120>  Anti-CD117 antibodies and methods of use thereof

<130>  BET18L2873

<160>  37

<170>  PatentIn version 3.5

<210>  1
<211>  8
<212>  PRT
<213>  Mus musculus

<400>  1

Gly Tyr Thr Phe Thr Arg Tyr Trp
1               5

<210>  2
<211>  8
<212>  PRT
<213>  Mus musculus

<400>  2

Ile Asp Pro Ser Asp Ser Glu Ile
1               5

<210>  3
<211>  13
<212>  PRT
<213>  Mus musculus

<400>  3

Ala Arg Asp Tyr Gly Ser Asp Tyr Tyr Ala Met Asp Tyr
1               5                   10

<210>  4
<211>  5
<212>  PRT
<213>  Mus musculus

<400>  4

Ser Ser Val Ser Tyr
1               5

<210>  5
<211>  7
<212>  PRT
<213>  Mus musculus

<400>  5

His Gln Trp Ser Ser Tyr Pro
1               5

<210>  6
<211>  5
<212>  PRT
<213>  Mus musculus

<400>  6

Arg Tyr Trp Met Asn
1               5

<210>  7
<211>  17
<212>  PRT
<213>  Mus musculus

<400>  7

Arg Ile Asp Pro Ser Asp Ser Glu Ile His Tyr Asn Gln Asn Phe Lys
1               5                   10                  15

Asp

<210>  8
<211>  11
<212>  PRT
<213>  Mus musculus

<400>  8

Asp Tyr Gly Ser Asp Tyr Tyr Ala Met Asp Tyr
1               5                   10

<210>  9
<211>  10
<212>  PRT
<213>  Mus musculus

<400>  9

Ser Ala Ser Ser Ser Val Ser Tyr Met His
1               5                   10

<210>  10
<211>  7
<212>  PRT
<213>  Mus musculus

<400>  10

Ser Thr Ser Asn Leu Thr Ser
1               5

<210>  11
<211>  7
<212>  PRT
<213>  Mus musculus

<400>  11

Gly Tyr Thr Phe Thr Arg Tyr
1               5


<210>  12
<211>  6
<212>  PRT
<213>  Mus musculus

<400>  12

Asp Pro Ser Asp Ser Glu
1               5


<210>  13
<211>  120
<212>  PRT
<213>  Mus musculus

<400>  13

Gln Val Gln Leu Gln Gln Ser Gly Ala Glu Leu Val Lys Pro Gly Ala
1               5                   10                  15

Pro Val Lys Leu Ser Cys Lys Ala Ser Gly Tyr Thr Phe Thr Arg Tyr
            20                  25                  30

Trp Met Asn Trp Val Lys Gln Arg Pro Gly Arg Gly Leu Glu Trp Ile
        35                  40                  45

Gly Arg Ile Asp Pro Ser Asp Ser Glu Ile His Tyr Asn Gln Asn Phe
    50                  55                  60

Lys Asp Lys Ala Thr Leu Thr Val Asp Lys Ser Ser Ser Thr Ala Tyr
65                  70                  75                  80

Ile Gln Leu Ser Ser Leu Thr Ser Glu Asp Ser Ala Val Tyr Tyr Cys
                85                  90                  95

Ala Arg Asp Tyr Gly Ser Asp Tyr Tyr Ala Met Asp Tyr Trp Gly Gln
            100                 105                 110

Gly Thr Ser Val Thr Val Ser Ser
        115                 120


<210>  14
<211>  360

<212>  DNA
<213>  Mus musculus

<400>  14
caggtccaac tgcagcagtc tggggctgag cttgtgaagc ctggggctcc agtgaagctg      60

tcctgcaagg cttctggcta caccttcacc agatactgga tgaactgggt gaagcagagg     120

cctggacgag gcctcgagtg gattggaagg attgatcctt ccgatagtga aattcattac     180

aatcaaaact tcaaggacaa ggccacactg actgtagaca aatcctccag cacagcctac     240

atccaactca gcagcctgac atctgaggac tctgcggtct attactgtgc aagagactat     300

ggatcggatt actatgctat ggactactgg ggtcaaggaa cctcggtcac cgtctcctca     360


<210>  15
<211>  104
<212>  PRT
<213>  Mus musculus

<400>  15

Gln Ile Val Leu Thr Gln Ser Pro Ala Ile Met Ser Ala Ser Leu Gly
1               5                   10                  15


Glu Glu Ile Thr Leu Thr Cys Ser Ala Ser Ser Ser Val Ser Tyr Met
            20                  25                  30


His Trp Tyr Gln Gln Lys Ser Gly Thr Ser Pro Lys Leu Leu Ile Tyr
        35                  40                  45


Ser Thr Ser Asn Leu Thr Ser Gly Val Pro Ser Arg Phe Ser Gly Ser
    50                  55                  60


Gly Ser Gly Thr Phe Tyr Ser Leu Thr Ile Ser Ser Val Glu Ala Glu
65                  70                  75                  80


Asp Ala Ala Asp Tyr Tyr Cys His Gln Trp Ser Ser Tyr Pro Phe Gly
                85                  90                  95


Gly Gly Thr Lys Leu Glu Ile Lys
            100


<210>  16
<211>  312
<212>  DNA
<213>  Mus musculus

<400>  16
caaattgttc tcacccagtc tccagcaatc atgtctgcat ctctagggga ggagattacc      60

ctaacctgca gtgccagctc gagtgtaagt tacatgcact ggtaccagca gaagtcaggc     120

acttctccca aactcttgat ttatagcaca tccaacctga cttctggagt cccttctcgc     180

ttcagtggca gtgggtctgg gaccttttat tctctcacaa tcagcagtgt ggaggctgaa 240

gatgctgccg attattactg ccatcagtgg agtagttatc cgttcggtgg aggcaccaag 300

ctcgagatca aa 312

<210> 17
<211> 330
<212> PRT
<213> Homo sapiens

<400> 17

<210>  18
<211>  990
<212>  DNA
<213>  Homo sapiens

<400>  18
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtcgtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 19
<211> 107
<212> PRT
<213> Homo sapiens

<400> 19

<210> 20
<211> 321
<212> DNA
<213> Homo sapiens

<400> 20
cgaactgtgg ctgcaccatc tgtcttcatc ttcccgccat ctgatgagca gttgaaatct 60

ggaactgcct ctgttgtgtg cctgctgaat aacttctatc ccagagaggc caaagtacag 120

tggaaggtgg ataacgccct ccaatcgggt aactcccagg agagtgtcac agagcaggac 180

agcaaggaca gcacctacag cctcagcagc accctgacgc tgagcaaagc agactacgag 240

aaacacaaag tctacgcctg cgaagtcacc catcagggcc tgagctcgcc cgtcacaaag 300

agcttcaaca ggggagagtg t 321

<210> 21
<211> 450
<212> PRT
<213> Artificial Sequence

<220>
<223> Chimeric Heavy Chain

<400> 21

Gln Val Gln Leu Gln Gln Ser Gly Ala Glu Leu Val Lys Pro Gly Ala
1               5                   10                  15

Pro Val Lys Leu Ser Cys Lys Ala Ser Gly Tyr Thr Phe Thr Arg Tyr
            20                  25                  30

Trp Met Asn Trp Val Lys Gln Arg Pro Gly Arg Gly Leu Glu Trp Ile
        35                  40                  45

Gly Arg Ile Asp Pro Ser Asp Ser Glu Ile His Tyr Asn Gln Asn Phe
    50                  55                  60

Lys Asp Lys Ala Thr Leu Thr Val Asp Lys Ser Ser Ser Thr Ala Tyr
65                  70                  75                  80

Ile Gln Leu Ser Ser Leu Thr Ser Glu Asp Ser Ala Val Tyr Tyr Cys
                85                  90                  95

Ala Arg Asp Tyr Gly Ser Asp Tyr Tyr Ala Met Asp Tyr Trp Gly Gln
            100                 105                 110

Gly Thr Ser Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450


<210>  22
<211>  1353
<212>  DNA
<213>  Artificial Sequence

<220>
<223>  Chimeric Heavy Chain

<400>  22
caggtccaac tgcagcagtc tggggctgag cttgtgaagc ctggggctcc agtgaagctg      60

tcctgcaagg cttctggcta caccttcacc agatactgga tgaactgggt gaagcagagg     120

cctggacgag gcctcgagtg gattggaagg attgatcctt ccgatagtga aattcattac     180

aatcaaaact tcaaggacaa ggccacactg actgtagaca aatcctccag cacagcctac     240

atccaactca gcagcctgac atctgaggac tctgcggtct attactgtgc aagagactat     300

ggatcggatt actatgctat ggactactgg ggtcaaggaa cctcggtcac cgtctcctca     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtcgtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag     960

gagtacaagt gcaaggtctc caacaaagcc ctcccagccc ccatcgagaa aaccatctcc    1020

aaagccaaag ggcagccccg agaaccacag gtgtacaccc tgcccccatc ccgggatgag    1080

ctgaccaaga accaggtcag cctgacctgc ctggtcaaag gcttctatcc cagcgacatc    1140

gccgtggagt gggagagcaa tgggcagccg gagaacaact acaagaccac gcctcccgtg    1200

ctggactccg acggctcctt cttcctctac agcaagctca ccgtggacaa gagcaggtgg    1260

cagcagggga acgtcttctc atgctccgtg atgcatgagg ctctgcacaa ccactacacg    1320

cagaagagcc tctccctgtc tccgggtaaa tga                                 1353


<210>  23
<211>  211

<212>  PRT
<213>  Artificial Sequence

<220>
<223>  Chimeric Light Chain

<400>  23

Gln Ile Val Leu Thr Gln Ser Pro Ala Ile Met Ser Ala Ser Leu Gly
1               5                   10                  15

Glu Glu Ile Thr Leu Thr Cys Ser Ala Ser Ser Ser Val Ser Tyr Met
            20                  25                  30

His Trp Tyr Gln Gln Lys Ser Gly Thr Ser Pro Lys Leu Leu Ile Tyr
        35                  40                  45

Ser Thr Ser Asn Leu Thr Ser Gly Val Pro Ser Arg Phe Ser Gly Ser
    50                  55                  60

Gly Ser Gly Thr Phe Tyr Ser Leu Thr Ile Ser Ser Val Glu Ala Glu
65                  70                  75                  80

Asp Ala Ala Asp Tyr Tyr Cys His Gln Trp Ser Ser Tyr Pro Phe Gly
                85                  90                  95

Gly Gly Thr Lys Leu Glu Ile Lys Arg Thr Val Ala Ala Pro Ser Val
            100                 105                 110

Phe Ile Phe Pro Pro Ser Asp Glu Gln Leu Lys Ser Gly Thr Ala Ser
        115                 120                 125

Val Val Cys Leu Leu Asn Asn Phe Tyr Pro Arg Glu Ala Lys Val Gln
    130                 135                 140

Trp Lys Val Asp Asn Ala Leu Gln Ser Gly Asn Ser Gln Glu Ser Val
145                 150                 155                 160

Thr Glu Gln Asp Ser Lys Asp Ser Thr Tyr Ser Leu Ser Ser Thr Leu
                165                 170                 175

Thr Leu Ser Lys Ala Asp Tyr Glu Lys His Lys Val Tyr Ala Cys Glu
            180                 185                 190

Val Thr His Gln Gly Leu Ser Ser Pro Val Thr Lys Ser Phe Asn Arg
        195                 200                 205

Gly Glu Cys
    210

<210>  24
<211>  633
<212>  DNA
<213>  Artificial Sequence

<220>
<223>  Chimeric Light Chain coding sequence

<400>  24
caaattgttc tcacccagtc tccagcaatc atgtctgcat ctctagggga ggagattacc      60

ctaacctgca gtgccagctc gagtgtaagt tacatgcact ggtaccagca gaagtcaggc     120

acttctccca aactcttgat ttatagcaca tccaacctga cttctggagt cccttctcgc     180

ttcagtggca gtgggtctgg gaccttttat tctctcacaa tcagcagtgt ggaggctgaa     240

gatgctgccg attattactg ccatcagtgg agtagttatc cgttcggtgg aggcaccaag     300

ctcgagatca aacgaactgt ggctgcacca tctgtcttca tcttcccgcc atctgatgag     360

cagttgaaat ctggaactgc ctctgttgtg tgcctgctga ataacttcta tcccagagag     420

gccaaagtac agtggaaggt ggataacgcc ctccaatcgg gtaactccca ggagagtgtc     480

acagagcagg acagcaagga cagcacctac agcctcagca gcaccctgac gctgagcaaa     540

gcagactacg agaaacacaa agtctacgcc tgcgaagtca cccatcaggg cctgagctcg     600

cccgtcacaa agagcttcaa caggggagag tgt                                  633


<210>  25
<211>  120
<212>  PRT
<213>  Artificial sequence

<220>
<223>  Humanized VH

<400>  25

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser Tyr
            20                  25                  30


Trp Met Asn Trp Val Arg Gln Ala Pro Gly Gln Gly Leu Glu Trp Met
        35                  40                  45


Gly Arg Ile Asp Pro Ser Asp Ser Glu Ile His Tyr Asn Gln Lys Phe
    50                  55                  60


Lys Asp Lys Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Tyr Gly Ser Asp Tyr Tyr Ala Met Asp Tyr Trp Gly Gln
            100                 105                 110

Gly Thr Leu Val Thr Val Ser Ser
        115                 120

<210>  26
<211>  360
<212>  DNA
<213>  Artificial sequence

<220>
<223>  Humanized VH

<400>  26
caggtacaat tggtccaaag tggtgcagaa gtgaaaaagc caggagcctc tgtcaaggtt      60

agttgtaaag cttcaggtta cacctttaca agttactgga tgaactgggt tagacaggca     120

cccggccaag gcctggagtg gatgggccgc atcgacccct ccgacagcga gattcactac     180

aaccagaagt tcaaggacaa agccactctc accgtcgata agagcacatc taccgcttat     240

atggagctga gcagtctccg tagtgaagat acagcagtgt actattgcgc aagagactac     300

ggaagcgact actatgccat ggactattgg ggccagggca cattggtcac tgtgagtagc     360

<210>  27
<211>  104
<212>  PRT
<213>  Artificial sequence

<220>
<223>  Humanized VL

<400>  27

Gln Ile Val Leu Thr Gln Ser Pro Ala Phe Gln Ser Val Thr Pro Gly
1               5                   10                  15

Glu Lys Val Thr Ile Thr Cys Ser Ala Ser Gln Ser Val Ser Tyr Met
            20                  25                  30

His Trp Tyr Gln Gln Lys Ser Gly Thr Ser Pro Lys Leu Leu Ile Tyr
        35                  40                  45

Ser Thr Ser Asn Leu Thr Ser Gly Val Pro Ser Arg Phe Ser Gly Ser
    50                  55                  60

Gly Ser Gly Thr Asp Tyr Thr Leu Thr Ile Ser Ser Leu Glu Ala Glu
65                  70                  75                  80

Asp Ala Ala Thr Tyr Tyr Cys His Gln Trp Ser Ser Tyr Pro Phe Gly
                85                  90                  95

Gly Gly Thr Lys Val Glu Ile Lys
            100

<210>  28
<211>  312
<212>  DNA
<213>  Artificial sequence

<220>
<223>  Humanized VL

<400>  28
caaattgtac tgacccaaag tccagcattc cagagtgtaa cacccggtga gaaggtcaca      60

attacctgct ctgcttccca atccgtgtcc tacatgcact ggtatcagca gaagtcaggc     120

acaagcccaa aactgctcat atactctacc agcaatctga cttcaggagt tccttcccga     180

ttttccggca gcggctccgg caccgactac acactgacca tctcatcttt ggaggccgag     240

gacgccgcta cctactactg tcaccagtgg agcagctatc ccttcggcgg tggtacaaag     300

gtggaaatca ag                                                         312

<210>  29
<211>  120
<212>  PRT
<213>  Artificial sequence

<220>
<223>  Humanized VH

<400>  29

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Trp Met Asn Trp Val Arg Gln Ala Pro Gly Gln Gly Leu Glu Trp Met
        35                  40                  45

Gly Arg Ile Asp Pro Ser Asp Ser Glu Ile His Tyr Asn Gln Lys Phe
    50                  55                  60

Lys Gly Arg Val Thr Met Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Tyr Gly Ser Asp Tyr Tyr Ala Met Asp Tyr Trp Gly Gln
            100                 105                 110

Gly Thr Leu Val Thr Val Ser Ser
        115                 120

<210>  30
<211>  360
<212>  DNA
<213>  Artificial sequence

<220>
<223>  Humanized VH

<400>  30
caggtacaat tggtccaaag tggtgcagaa gtgaaaaagc caggagcctc tgtcaaggtt      60

agttgtaaag cttcaggtta cacctttaca agttactgga tgaactgggt tagacaggca     120

cccggccaag gcctggagtg gatgggccgc atcgacccct ccgacagcga gattcactac     180

aaccagaagt tcaagggcag agtcactatg accgtcgata caagcacatc taccgcttat     240

atggagctga gcagtctccg tagtgaagat acagcagtgt actattgcgc aagagactac     300

ggaagcgact actatgccat ggactattgg ggccagggca cattggtcac tgtgagtagc     360

<210>  31
<211>  120
<212>  PRT
<213>  Artificial sequence

<220>
<223>  Humanized VH

<400>  31

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Trp Met Asn Trp Val Arg Gln Ala Pro Gly Gln Gly Leu Glu Trp Ile
        35                  40                  45

Gly Arg Ile Asp Pro Ser Asp Ser Glu Ile His Tyr Asn Gln Lys Phe
    50                  55                  60

Lys Gly Arg Val Thr Met Thr Val Asp Thr Ser Thr Ser Thr Ala Tyr
65                  70                  75                  80

Met Glu Leu Ser Ser Leu Arg Ser Glu Asp Thr Ala Val Tyr Tyr Cys

85                  90                  95

Ala Arg Asp Tyr Gly Ser Asp Tyr Tyr Ala Met Asp Tyr Trp Gly Gln
            100                 105                 110

Gly Thr Leu Val Thr Val Ser Ser
        115                 120

<210>  32
<211>  360
<212>  DNA
<213>  Artificial sequence

<220>
<223>  Humanized VH

<400>  32
caggtacaat tggtccaaag tggtgcagaa gtgaaaaagc caggagcctc tgtcaaggtt      60

agttgtaaag cttcaggtta cacctttaca agttactgga tgaactgggt tagacaggca     120

cccggccaag gcctggagtg gattggccgc atcgacccct ccgacagcga gattcactac     180

aaccagaagt tcaagggcag agtcactatg accgtcgata caagcacatc taccgcttat     240

atggagctga gcagtctccg tagtgaagat acagcagtgt actattgcgc aagagactac     300

ggaagcgact actatgccat ggactattgg ggccagggca cattggtcac tgtgagtagc     360

<210>  33
<211>  8
<212>  PRT
<213>  artificial sequence

<220>
<223>  Hz-H-CDR1

<400>  33

Gly Tyr Thr Phe Thr Ser Tyr Trp
1               5

<210>  34
<211>  5
<212>  PRT
<213>  artificial sequence

<220>
<223>  Hz-H-CDR1

<400>  34

Ser Tyr Trp Met Asn
1               5

<210>  35
<211>  7

<212>  PRT
<213>  artificial sequence

<220>
<223>  Hz-H-CDR1

<400>  35

Gly Tyr Thr Phe Thr Ser Tyr
1               5


<210>  36
<211>  5
<212>  PRT
<213>  artificial sequence

<220>
<223>  Hz-L-CDR1

<400>  36

Gln Ser Val Ser Tyr
1               5


<210>  37
<211>  10
<212>  PRT
<213>  artificial sequence

<220>
<223>  Hz-L-CDR1

<400>  37

Ser Ala Ser Gln Ser Val Ser Tyr Met His
1               5                   10